# EUROPEAN PATENT SPECIFICATION

(11) **EP 1 866 650 B1**
(45) Date of publication and mention of the grant of the patent: **19.05.2010**
(21) Application number: 06723873.3
(22) Date of filing: 30.03.2006
(51) Int. Cl.: G01N 33/68, G01N 33/94

(54) **NEURODEGENERATIVE MARKERS FOR DEPRESSION.**
NEURODEGENERATIVE MARKER FÜR DEPRESSION.
MARQUEURS NEURODÉGÉNÉRATIFS POUR DÉPRESSION.

(30) Priority: 06.04.2005 EP 05007558
(43) Date of publication of application: 19.12.2007
(60) Divisional application: 09013088.1
(73) Proprietor: Verstappen, Leopold, 2570 Duffel (BE); University of Maastricht, 6200 MD Maastricht (NL); University of Antwerp, 2610 Wilrijk (BE); Klinik und Poliklinik für Psychiatrie und Psychotherapie, 80336 München (DE)
(72) Inventor: MYINT, Aye, Mu, B-2300 Turnhout (BE); SCHAWALLER, Manfred, CH-1785 Cressier (CH); VERKERK, Robert, B-2640 Mortsel (BE); SCHWARZ, Markus, J., 81373 München (DE); MÜLLER, Norbert, 80686 München (DE)
(74) Representative: Müller-Boré & Partner Patentanwälte
(86) International application number: PCT/EP2006/002907
(87) International publication number: WO 2006/105907

(56) References cited:
- WADA H ET AL: "[Concentrations of multiple neurochemicals in the cerebrospinal fluid of patients with senile dementia and the relationship to alpha 1-antichymotrypsin]" NIPPON RONEN IGAKKAI ZASSHI. JAPANESE JOURNAL OF GERIATRICS. JAN 1993, vol. 30, no. 1, January 1993 (1993-01), pages 46-53, XP008064388 ISSN: 0300-9173
- AMIRKHANI ARDESHIR ET AL: "Quantitation of tryptophan, kynurenine and kynurenic acid in human plasma by capillary liquid chromatography-electrospray ionization tandem mass spectrometry." JOURNAL OF CHROMATOGRAPHY B, vol. 780, no. 2, 25 November 2002 (2002-11-25), pages 381-387, XP002381686 ISSN: 1387-2273
- FEVRE-MONTANGE M: "[Melatonin]" PRESSE MÉDICALE (PARIS, FRANCE : 1983) 21 SEP 1985, vol. 14, no. 31, 21 September 1985 (1985-09-21), pages 1659-1663, XP008064358 ISSN: 0755-4982
- SJÖSTRÖM R: "Diagnosis of manic-depressive psychosis from cerebrospinal fluid concentration of 5-hydroxyindoleacetic acid." ADVANCES IN BIOCHEMICAL PSYCHOPHARMACOLOGY. 1974, vol. 11, no. 0, 1974, pages 369-375, XP008064376 ISSN: 0065-2229
- ANDERSON I M ET AL: "DECREASED PLASMA TRYPTOPHAN CONCENTRATION IN MAJOR DEPRESSION RELATIONSHIP TO MELANCHOLIA AND WEIGHT LOSS" JOURNAL OF AFFECTIVE DISORDERS, vol. 20, no. 3, 1990, pages 185-192, XP002381687 ISSN: 0165-0327
- WIDNER B ET AL: "Tryptophan degradation and immune activation in Alzheimer's disease" JOURNAL OF NEURAL TRANSMISSION, vol. 107, no. 3, 15 March 2000 (2000-03-15), pages 343-353, XP002381688 ISSN: 0300-9564
- PERRET G ET AL: "[L-tryptophan plasma levels in treatment resistant depressive states]" REVUE MÉDICALE DE LA SUISSE ROMANDE. FEB 2000, vol. 120, no. 2, February 2000 (2000-02), pages 153-157, XP008064360 ISSN: 0035-3655
- HEYES M P ET AL: "Quinolinic acid and kynurenine pathway metabolism in inflammatory and non-inflammatory neurological disease." BRAIN : A JOURNAL OF NEUROLOGY. OCT 1992, vol. 115 ( Pt 5), October 1992 (1992-10), pages 1249-1273, XP008064389 ISSN: 0006-8950
- SALZMAN C. ET AL: 'Textbook of Psychopharmacology, Second Edition, Edited by Alan F. Schatzberg and Charles B. Nemeroff', 1998, AMEERICAN PSYCHIATRIC PRESS, INC., WASHINGTON DC, USA * page 970 - page 971 *
- TASKFORCE ON DSM-IV: "Diagnostic and Statistical Manual of Mental Disorders, Fourth Edition, Text Revision" 2000, AMERICAL PSYCHIATRIC ASSOCIATION , WASHINGTON DC, USA * page 154 - page 158 *
- AYE-MU MYINT ET AL: 'Kynurenine pathway in major depression: Evidence of impaired neuroprotection' JOURNAL OF AFFECTIVE DISORDERS vol. 98, 2007, pages 143 - 151
- AYE MU MYINT ET AL: 'Change of the balances between kynurenine metabolites in medication-naive and medication-free schizophrenic patients' IN PRESS
- 'Kynurenine metabolites in schizophrenia' SUPPLEMENT (1)
- 'Kynurenine metabolites in serum and cerebrospinal fluid of patients with major psychiatric disorders' SUPPLEMENT (3)
- BERKOW R.; FLETCHER A.J.: 'The Merck Manual of Diagnosis and Therapy, Fifteenth Edition', 1987, MERCK SHARP & DOME RESEARCH LABORATORIES, NEW YORK, USA * page 1455, paragraph 12 *

## Description

The present invention relates to methods for detecting depression comprising the steps of measuring the concentration of at least one *in vivo* degradation product of tryptophan. Further, the present invention relates to the use of said values as predictive markers for the detection of depression.

Depression is a major psychiatric disorder with an overall incidence of 12.3% with 14.1% for female and 8.6% for male in Europe (Copeland JR, Beekman AT, Dewey ME, Hooijer C, 1999, Depression in Europe. Geographical distribution among older people. Br. J. Psychiatry; 174:312-321). In the community and among employees, there are cases of undiagnosed depression. In America, at any time, 1 in 20 employees can experience depression and if it is left untreated, depression leads to a decrease of productivity and an increase of sick days. According to National Mental Health Association, American employees took around three million days off work every year due to untreated depression and that is more than employees used for other physical illnesses like diabetes, high blood pressure and arthritis (Burns H, Charleston Regional Business Journal, April, 2004).

To solve the problems with undiagnosed or under-diagnosed depression in the community, home care nurses were assigned to perform interviews using structured format such as PRIME-MD test or PDI-29 test, both of which are made to assess the psychological status of a person. The sensitivity of PRIME-MD is 41.7% and specificity is 83% whereas the PDI-29 test which is considered to be a better test reaches the sensitivity of 73.6% and the specificity of 59% (Preville M, Cote G, Boyer R, Hebert R (2004). Detection of depression and anxiety disorders by home care nurses. Aging Ment. Health; 8(5): 400-409).

There are theories regarding the roles of neurochemicals in depression. The group of neurochemicals found to be important in the pathophysiology of depression was monoamine and was proposed since 1960s (Coppen A, 1969, Defects in monoamine metabolism and their possible importance in the pathogenesis of depressive syndrome. Psychiatr. Neural Neurochir; 72(2): 173-180). Moreover, it was found that tryptophan gave added effect to monoamine-oxidase inhibitors, the antidepressants (Coppen A and Noguera R, 1970, L-tryptophan in depression. Lancet; 1(7656):1111). Few years later, the role of serotonin (5HT) was proposed in pathogenesis of affective disorders (Coppen A and Wook K, 1982, 5-Hydroxytryptamine in the pathogenesis of effective disorders. Adv. Biochem. Psychopharmacol. 34: 249-258).

Alzheimer's disease (AD) is the most frequent neurodegenerative disorder of the human brain. Especially in early stages of AD, it is important, but difficult to differentiate between depression accompanied with subjective cognitive impairment (so-called pseudodementia or dementia syndrome of depression) and AD (Tekin,S. and Cummings,J.L. (2001). Depression in dementia. Neurologist 7, 252-259). The prevalence of depression ranges between 15 and 50% in patients with AD (Rovner,B.W., Broadhead,J., Spencer,M., Carson,K., and Folstein,M.F. (1989). Depression and Alzheimer's disease. Am. J. Psychiatry 146, 350-353.; Migliorelli,R., Teson,A., Sabe,L., Petracchi,M., Leiguarda,R., and Starkstein,S.E. (1995). Prevalence and correlates of dysthymia and major depression among patients with Alzheimer's disease. Am. J. Psychiatry 152, 37-44) and several authors suggested that depressive symptoms are part of the preclinical phase of AD ( Berger,A.K., Fratiglioni,L., Forsell,Y., Winblad,B., and Backman,L. (1999). The occurrence of depressive symptoms in the preclinical phase of AD: a population-based study. Neurology 53, 1998-2002.; Visser,P.J., Verhey,F.R., Ponds,R.W., Kester,A., and Jolles,J. (2000). Distinction between preclinical Alzheimer's disease and depression. J. Am. Geriatr. Soc. 48, 479-484.).

Serotonin is a neurochemical which is necessary for the brain for the good mood and growth factors for the brain, and which is synthesized from tryptophan. Tryptophan is an amino acid from the food and from the body amino acid pool. Tryptophan is partly broken down by an enzyme, indoleamine 2,3-dioxygenase, which is present in the lungs, white blood cells, placenta and the brain (Heyes MP, Satio K, Markey SP, 1992, Human macrophages convert L-tryptophan into the neurotoxin quinolinic acid. Biochem. J.; 283(3):633-635; Mellor AL and Munn DH, 1999, Tryptophan catabolism and T-cell tolerance: immunosuppression by starvation. Immunol. Today; 20(10):469-473) and partly broken down by the tryptophan-dioxygenase in the liver. The tryptophan catabolic pathway or kynurenine pathway through indoleamine-2,3-digoxygenase exists both in the blood and in the brain and 60% of brain kynurenines come from the peripheral blood (Gal EM, Sherman AD, 1980, L-Kynurenine: synthesis and possible regulatory function in brain. Neurochem Res; 5(3): 223-239).

When tryptophan is degraded through the kynurenine pathway, the next product is kynurenine which is the first metabolite of tryptophan (Bender DA, 1989, The kynurenine pathway of tryptophan metabolism: In TW Stone (ed). Quinolinic acid and kynurenines. Boca Raton FL: CRC Press: 3-38). This kynurenine is again broken down into two pathways: (1) neuroprotective, kynurenic acid and (2) neurodegenerative 3-hydroxykynurenine (3-HK), hydroxyanthranilic acid and quinolinic acid (Chiarugi A, Calvani M, Meli E, Traggiai E, Moroni F, 2001, Synthesis and release of neurotoxic kynurenine metabolites by human monocyte derived macrophages. J Neuroimmunol;120(1-2):190-198). Normally, formation of quinolinic acid is faster and kynurenic acid has a counteractive protective role against quinolinic acid (Perkins MN and Stone TW, 1982, An iontophoretic investigation of the action of convulsant kynurenines and their interaction with the endogenous excitant quinolinic acid. Brain Res.;247(1):184-187). Based on the above evidences, a hypothesis was proposed that the imbalance in neurodegenerative neuroprotective pathways bring a person to chronically depressed state (Myint AM and Kim YK, 2003, Cytokine-serotonin interaction through IDO: a neurodegeneration hypothesis of depression. Medical Hypothesis; 61 (5-6): 519-525).

Further, Wada et al. (Japanese Journal of Geriatrics, 30(1), January 1993, pp. 46-53) describes the concentrations of multiple neurochemicals in the lumbar cerebrospinal fluid of patients with and without senile dementia.

Further, Amirkhani et al. (Journal of Chromatography B, 780(2), November 2002, pp. 381-387) describes the quantitation of tryptophan, kynurenine, and kynurenic acid in human plasma by capillary liquid chromatography-electrospray ionization tandem mass spectrometry.

Further, Fevre-Montagne (Presse Médicale, 14(31), September 1985, pp. 1659-1663) describes the physiology of the epiphyseal hormone melatonin.

Further, Sjöström (Advances in Biochemical Psychopharmacology, 11, 1974, pp. 369-375) describes the use of the measurement of acidic monoamine metabolites in cerebrospinal fluid in the diagnosis of manic-depressive psychosis.

Further, Anderson et al. (Journal of Affective Disorders, 20(3), 1990, pp. 185-192) describes decreases plasma tryptophan concentration in major depression and its relationship to melancholia and weight loss.

Maes and coworkers found that plasma kynurenine and the kynurenine/tryptophan quotient were significantly more pronounced in women whose anxiety and depression scores significantly increased in the puerperium (Maes, M. et. al., Life Sciences 71 (2002), pp 1837-1848).

Further, Widner et al. (Journal of Neural Transmission, 107(3), March 2000, pp. 343-35) describes tryptophan degradation and immune activation in Alzheimer's disease.

Furthermore, Perret et al. (Revue Médicale de la Suisse Romande, 120(2), February 2000, pp. 153-157) describes tryptophan plasma levels in treatment resistant depression.

Finally, Heyes et al. (Brain: A Journal of Neurology, 115(5), October 1992, pp. 1249-1273) describes quinolinic acid and kynurenine pathway metabolism in inflammatory and non-inflammatory neurological disease.

In the art depression has been diagnosed using protein markers such as those described in WO 02/057790.

Though scientists in the area of neuroscience tried to find out the aetiological factor for major depression, no single factor was found and depression is considered as a disease caused by both genetic or congenital and environmental factors such as psychological stress and certain chronic diseases such as cancer. In addition, biochemical diagnostic markers have been searched but no efficient biomarker was found.

Thus, the problem underlying the present invention is to provide a new method for detecting depression using low molecular weight biochemical markers.

The solution to the above technical problem is achieved by the embodiments characterized in the claims.

In particular, the present invention relates to a method for detecting depression comprising the step of measuring the concentration of at least one of the following *in vivo* degradation products of tryptophan, 3-hydroxykynurenine, kynurenic acid and/or kynurenine, in a blood plasma sample obtained from the individuum being examined, and assessing the psychiatric condition.

The blood plasma sample can be obtained by any method known in the art. In a preferred embodiment of the present invention an overnight-fasting early morning blood sample of a patient is collected in a heparinised tube. The blood plasma is obtained via centrifugation of said heparinised tube which results in a separation of the plasma fraction which forms the supernatant. In a more preferred embodiment the plasma is frozen, most preferably at -70°C, before measuring the concentration of at least one *in vivo* degradation product of tryptophan. Instead of blood plasma any other sources of body fluids such as whole blood, serum, urine, saliva and cerebrospinal fluid (CSF) can also be used in the present invention. These body fluids can be obtained by methods known in the art.

In a preferred embodiment of the present invention the above method further comprises the step of measuring the concentration of kynurenine in the blood plasma sample.

In another preferred embodiment of the present invention the above method further comprises the step of measuring the concentration of 3-hydroxykynurenine in the blood plasma sample.

The measurement of the concentration of tryptophan and/or degradation products such as 3-hydroxykynurenine and/or kynurenic acid and/or kynurenine in a body fluid such as a blood plasma sample can be carried out by any method known in the art. In a preferred embodiment of the present invention the measurement of the concentration of the analytes, especially the degradation products of tryptophan, is carried out using High Performance Liquid Chromatography, in a more preferred embodiment using a UV detector and/or a flourescent detector, and/or an immunoassay and/or a ligand binding assay. In a preferred embodiment of the present invention the analytes are determined by a ligand binding assay, for example an immunoassay based on antibodies or a receptor binding assay or an enzyme binding assay or competitive versions of one or more of those assays.

In another embodiment of the present invention, the samples are for example substantially deproteinated (all proteins are removed) before measuring the concentration of at least one of the following *in vivo* degradation products of tryptophan, 3-hydroxykynurenine, kynurenic acid, and/or kynurenine.

After measuring the concentrations of kynurenic acid and kynurenine in a blood sample the neuroprotective ratio may be determined by dividing the value of the concentration of kynurenic acid by the value of the concentration of kynurenine in said blood plasma sample (kynurenic acid value/kynurenine value).

In a preferred embodiment of the present invention an individuum having a depression is characterized by a neuroprotective ratio in the blood plasma of 0 to 18, more preferably 3 to 17, and most preferably 6 to 16.3.

Further, after measuring the concentrations of kynurenic acid and kynurenine in a blood sample the neuroprotective index may be determined by dividing the square value of the concentration of kynurenic acid by the value of the concentration of kynurenine in said blood plasma sample (kynurenic acid value²/kynurenine value).

In a preferred embodiment of the present invention an individuum having a depression is characterized by a neuroprotective index in the blood plasma of 0 to 700, more preferably 100 to 600, and most preferably 200 to 473.

In another embodiment of the present invention, after measuring the concentrations of kynurenic acid and 3-hydroxykynurenine in a blood sample the ratio ("neurodegenerative ratio") may be determined by dividing the value of the concentration of 3-hydroxykynurenine by the value of the concentration of kynurenic acid or of tryptophan (3-hydroxykynurenine value/kynurenic acid value or 3-hydroxykynurenine value/ tryptophan value).

In a preferred embodiment of the present invention the ratio determined by dividing the value of the concentration of 3-hydroxykynurenine by the value of the concentration of kynurenic acid is significantly increased when compared to healthy individuals.

In another preferred embodiment of the present invention an individuum having a depression is characterized by a ratio determined by dividing the value of the concentration of 3-hydroxykynurenine by the value of the concentration of kynurenic acid in the blood plasma. For example, if the ratio is about two or higher, provided that the analytes are given in the same unit, this is considered as an indication of depression. The same applies to the ratio determined by dividing the value of the concentration of 3-hydroxykynurenine multiplied by the factor 1000 by the value of the concentration of tryptophan in the blood plasma; i.e. 3-HK is multiplied by the factor 1000, provided that the analytes are given in the same unit (3-HK x 1000 / TRP).

Any mathematical formula known in the art that uses e.g. tryptophan, 3-hydroxykynurenine, kynurenine and kynurenic acid as input values and yields in the same result of interpretation may be used according to the present invention.

The present invention further relates to a method for detecting depression comprising the step of combining at least two values selected from the group consisting of the concentration of kynurenic acid, the neuroprotective ratio, the ratio determined by dividing the value of the concentration of 3-hydroxykynurenine by the value of the concentration of kynurenic acid, and the neuroprotective index of a blood plasma sample in order to improve the specificity and/or sensitivity of the detection of depression.

Methods for detecting depression comprising the measurement of at least one other neurodegenerative, neuroprotective, or neurotrophic marker(s) in combination with the measurement of the concentration of at least one of said *in vivo* degradation products of tryptophan are also part of the present invention.

Additionally, the present invention relates to (i) the use of a neuroprotective ratio determined by dividing the value of the concentration of kynurenic acid by the value of the concentration of kynurenine in a blood plasma sample as a predictive marker for the detection of depression, and/or (ii) the use of a ratio determined by dividing the value of the concentration of 3-hydroxykynurenine by the value of the concentration of kynurenic acid for the detection of depression, and/or (iii) the use of a neuroprotective index determined by dividing the square value of the concentration of kynurenic acid by the value of the concentration of kynurenine in a blood plasma sample as a predictive marker for the detection of depression.

The present invention also relates to the use of a combination of at least two values selected from the group consisting of the concentration of kynurenic acid, the neuroprotective ratio, the ratio determined by dividing the value of the concentration of 3-hydroxykynurenine by the value of the concentration of kynurenic acid and the neuroprotective index of a blood plasma as predictive markers for the detection of depression.

The present invention also relates to the use of a combination of the concentration of kynurenic acid, the neuroprotective ratio, the ratio determined by dividing the value of the concentration of 3-hydroxykynurenine by the value of the concentration of kynurenic acid, the neuroprotective index of a blood plasma, and/or at least one other neuroprotective, neurodegenerative or neurotrophic marker(s) as predictive markers for the detection of depression.

The terms "predictive marker" or "biological marker" as used herein means that the factor used as a biological or predictive marker, preferably the concentration of kynurenic acid in blood plasma, the neuroprotective ratio, the ratio determined by dividing the value of the concentration of 3-hydroxykynurenine by the value of the concentration of kynurenic acid, the neuroprotective index, or a combination thereof, is indicative regarding the question whether an individuum has a depression.

The present invention can be used in form of a kit for detecting a psychiatric condition containing means for detecting the concentration of a tryptophan degeneration product in a body fluid such as whole blood, serum, plasma, urine, saliva and CSF. .

In a preferred embodiment the kit contains means for detecting the concentration of kynurenic acid and/or kynurenine and/or 3-hydroxykynurenine and/or tryptophan in said blood plasma sample.

The present invention can be used for therapeutic interventions that change any of the above mentioned biomarkers.

The figures show:
Figure 1 shows the frequency of the kynurenic acid concentrations in nanomole/litre as obtained in Example 1.
Figure 2 shows the frequency of the neuroprotective ratio (KAKYN) as obtained in Example 1.
Figure 3 shows the frequency of the neuroprotective index (PROi) as obtained in Example 1.
Figure 4 shows the Receiver Operating Characteristic (ROC) curve for kynurenic acid (KA) as obtained in Example 1.
Figure 5 shows the ROC curve for the neuroprotective ratio (KAKYN) as obtained in Example 1.
Figure 6 shows the ROC curve for the neuroprotective index (PROi) as obtained in Example 1.

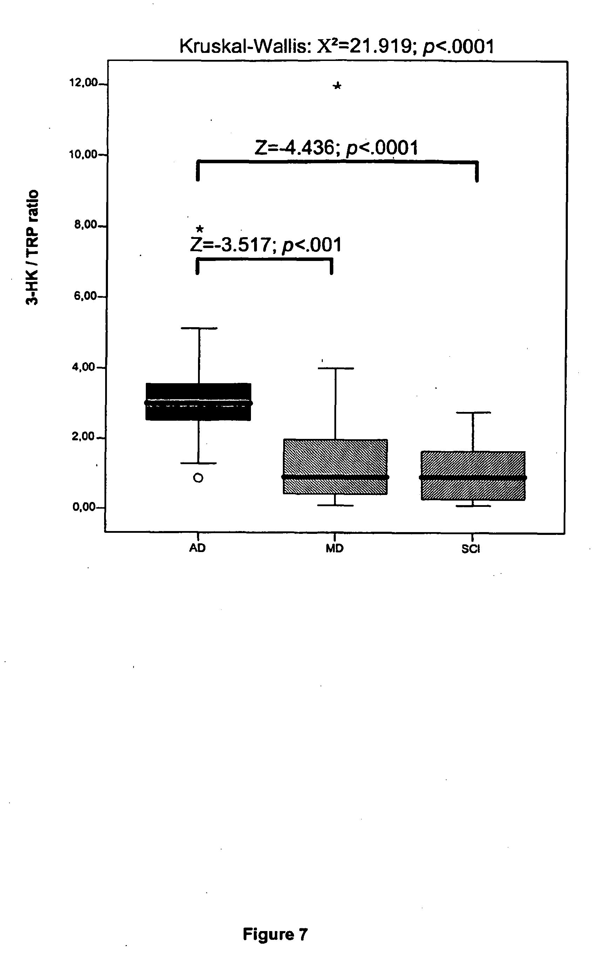

Figure 7 shows ratios between serum levels of 3-HK and TRP in patients with Alzheimers disease (AD), patients with major depression (MD), and healthy persons with subjective cognitive impairment (SCI). Kruskal-Wallis test revealed a significant difference between the three investigated groups, and Mann-Whitney test confirmed the significant difference between AD patients and the two comparison groups.

Table 1 shows the results of Example 1 (Gender; M = male, F = female; Age (years); TYR (tyrosine in micromole/litre); VAL (valine in micromole/litre); TRP (tryptophan in micromole/litre); PHE (phenylalanine in micromole/litre); ILE (isoleucine in micromole/litre); LEU (leucine in micromole/litre); Kyn (Kynurenine in micromole/litre); KA (Kynurenic acid in nanomole/litre); TRPi (tryptophan index = 100 x tryptophan/{TYR + VAL + PHE + ILE + LEU}); KYN/TRP (Tryptophan breakdown index = Kynurenine value/Tryptophan value); KAKYN (Neuroprotective ratio = 1000 x Kynurenic acid value (micromole/litre)/Kynurenine value (micromole/litre)); PROi (Neuroprotective index = 1,000,000 kynurenic acid value(micromole/litre) x kynurenic acid value(micromolellitre)/kynurenine value(micromole/litre)))
Table 2 shows group characteristics of patients and control subjects. AD = Patients with Alzheimer's disease; MD = Patients with major depression; SCI: Healthy persons with subjective cognitive impairment; MMSE = Mini-Mental State Examination.
Table 3 shows mobile phases and gradient conditions. Solvent A: 50 mM sodium acetate, pH 4.8; solvent B: 50 mM sodium acetate, pH 3.56; solvent C: 100% acetonitrile; solvent D: 100% methanol.
Table 4 shows serum levels of tryptophan (TRP), kynurenine (KYN), kynurenic acid (KYNA), and 3-hydroxykynurenine (3-HK) in patients with Alzheimer's disease (AD), patients with major depression (MD), and healthy persons with subjective cognitive impairment (SCI). The non-parametric Kruskal-Wallis test was used to test for differences between the three investigated groups. The significant differences regarding 3-HK levels and 3-HK/TRP ratio was confirmed by Mann-Whitney test.

The present invention will now be further illustrated in the following examples without being limited thereto.

### Examples

### Example 1: Measurement of neuroprotective index, neuroprotective ratio and plasma kynurenine concentrations in human blood plasma

### Subjects

A total of 48 depressed patients (age of 44.277 ± 11.42 years) who were drug naive at the time of admission to the psychiatric department of the general hospital were recruited. A total of 95 normal healthy persons (age of 31.63 ± 8.5 years) who came to the same general hospital for regular check-up during the same period of time were recruited as controls. Informed consent was taken from all the subjects.

All the patients were interviewed by a qualified psychiatrist and diagnosed as major depression according to DSM-IV criteria (American Psychiatric Association, 1994, Diagnostic and Statistical Manual of Mental Disorders - Fourth Edition (DSM IV). Am. Psy. Asso. Washington DC). All those patients with co-morbidity of other psychiatric disorders including alcoholism and other acute or chronic diseases were excluded. Presence of other diseases were checked by both clinically and biochemically.

All the controls were checked for being free of chronic and acute diseases in the same way as patients. The interview was done by a qualified psychiatrist to confirm whether they were free from psychiatric and related disorders.

### Sample collection

A total of 10 ml of overnight-fasting early morning blood samples were collected in heparinised tubes and plasma samples were collected and stored at -70°C for analyses at a later date. For the patients, another samplings were done at the time of discharge.

### Sample and data analysis

Samples were analysed for (1) tryptophan (µmol/l), (2) competing amino acids: tyrosine, valine, phenylalanine, isoleucine, leucine, taurine, a-amino-n-butyric acid, and methionine (µmol/l), (3) kynurenine (µmol/l), hydroxy-anthranilic acid (nmol/l), and (4) kyrenic acid (nmol/l), using High Performance Liquid Chromatography using UV detector and the flourescent detectors (Herve C, Beyne P, Jamault H, Delacoux E, 1996, Determination of tryptophan and its kynurenine pathway metabolites in human serum by high-performance liquid chromatography. J. Chromatography B: Biomedical applications; (675):157-161.).

The tryptophan index (TRPi) which represents the tryptophan availability in the blood (100 x tryptophan value/sum of competing amino acids values), tryptophan breakdown index (KYN/TRP) which represents the tryptophan breakdown (kynurenine value/tryptophan value), and neuroprotective ratio (KAKYN) (kynurenic acid value/kynurenine value) and neuroprotective index (PROi) (kynurenic acid value²/kynurenine value) which represent the strength of neuroprotection against quinolinic excitotoxic effect were calculated (Table 1).

Statistical analysis was done using SPSS version 11.0.

### Results

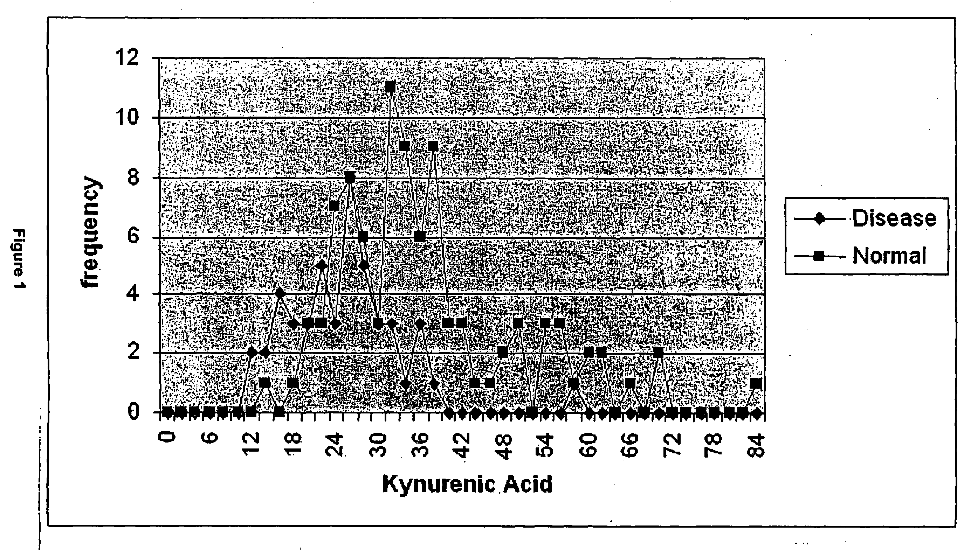

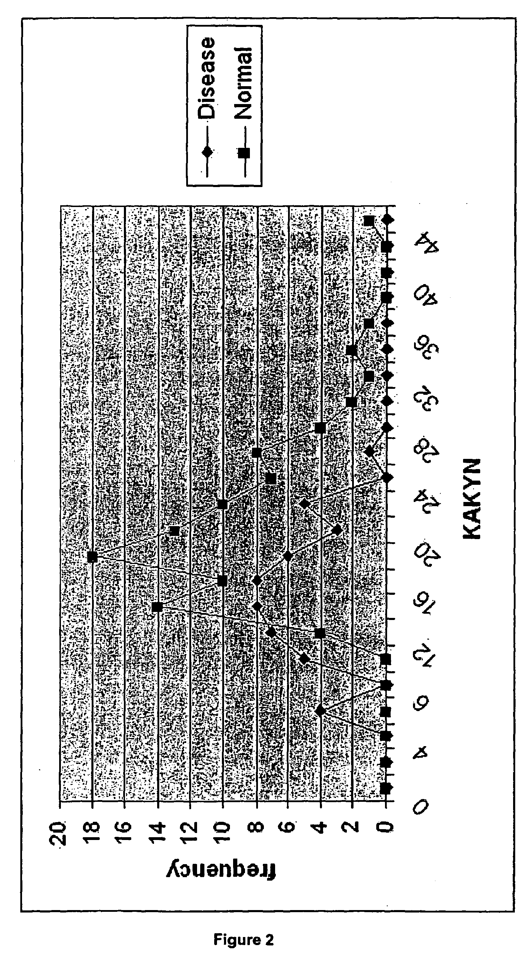

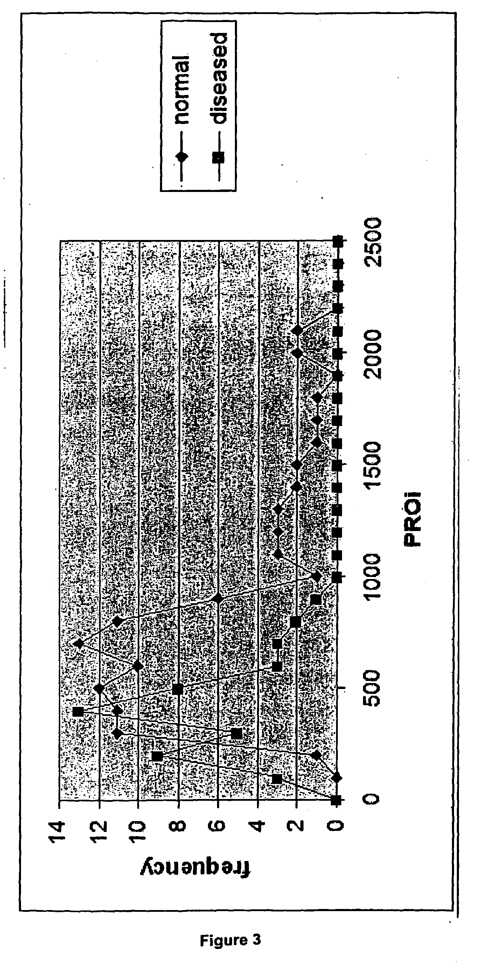

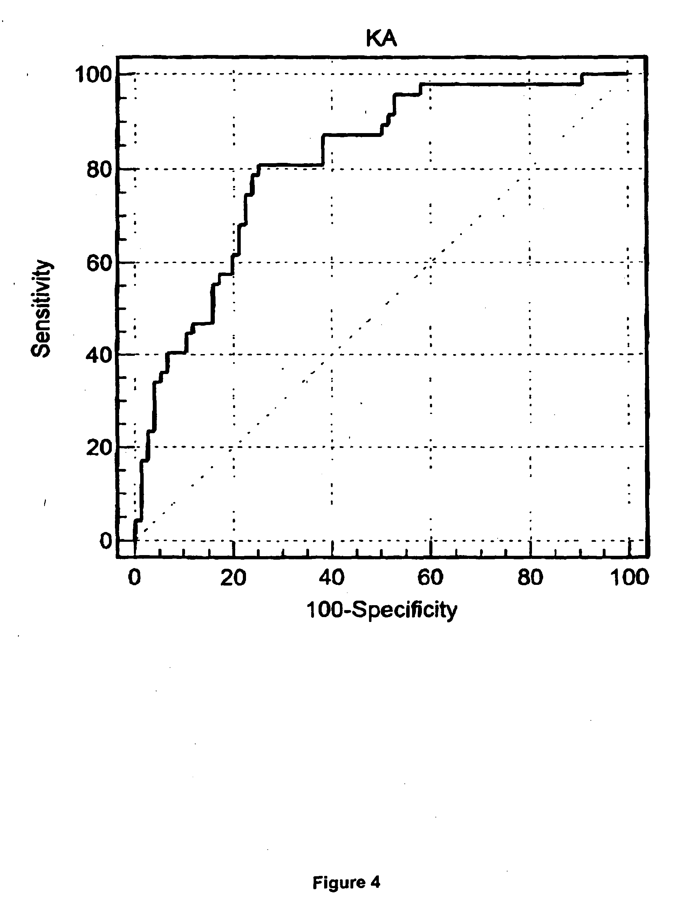

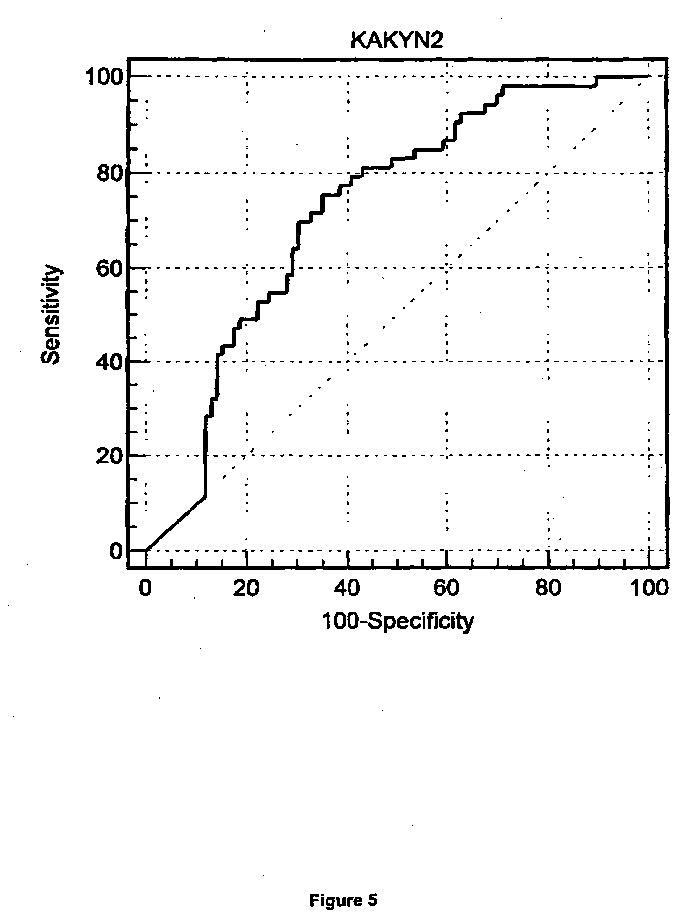

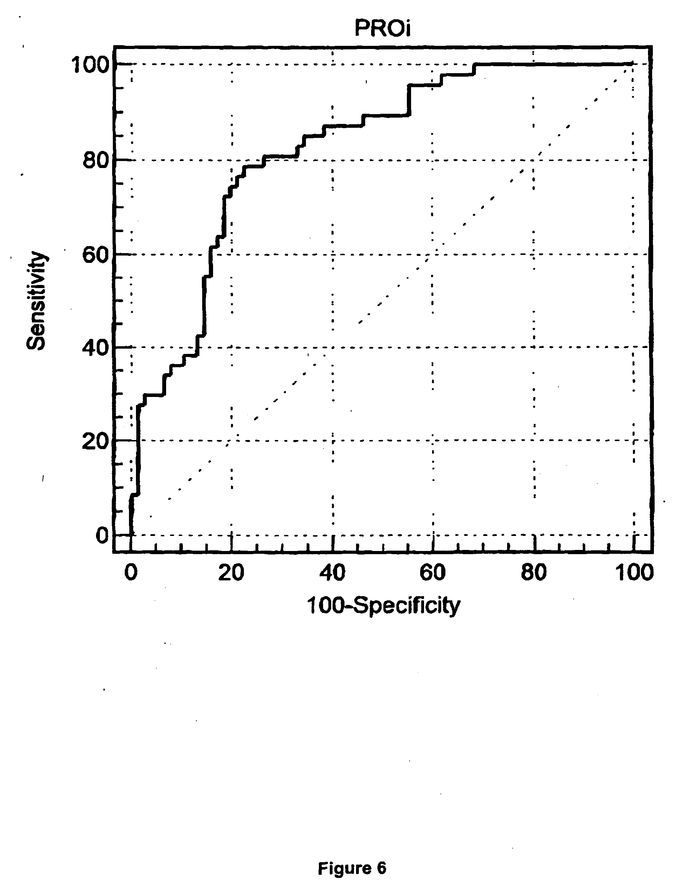

(1) On admission, the tryptophan index in the patients were significantly but not strongly lower (p<0.05) than the normal controls (9.99±0.26 vs 10.88±0.14).
(2) On admission, the tryptophan breakdown index in the patients were significantly but not strongly lower (p<0.04) than the normal controls (0.03±0.002 vs 0.05±0.02).
(3) On admission, the hydroxy-anthranilic acid (a step before formation of quinolinic acid in the catabolism) which indicates the toxic effect on the neurons showed no difference (p = 0.1) between patients (25.265 ± 2.437) and normal controls (25.182 ± 0.768).
(4) On admission, the plasma kynurenic acid concentrations in the patients were significantly and strongly lower (p<0.0001) than the normal controls (24.29±1.18 vs 35.96±1.37) (Figure 1).
(5) The ROC curve analysis showed that the kynurenic acid value 29.3 nmol/l is the cut-off point between depressed and normal with sensitivity of 80.9% and specificity of 75% (Figure 4).
(6) On admission, the neuroprotective ratio in the patients were significantly and strongly lower (p<0.0001) than the normal controls (14.08±0.64 vs 19.36±0.61) (Figure 2).
(7) The ROC curve analysis showed that the neuroprotective index 16.3 is the cut-off point between depressed and normal with sensitivity of 75.5% and specificity of 65% (Figure 5).
(8) On admission, the neuroprotective index in the patients were significantly lower (p<0.04) than the normal controls (358.77±52.93 vs 757.7±86.22) (Figure 3).
(9) The ROC curve analysis showed that the neuroprotective index 473 is the cut-off point between depressed and normal with sensitivity of 78.7% and specificity of 77.6% (Figure 6).
(10) The univariate analysis showed that the above three markers were not influenced by age or gender.

### Conclusion

The neuroprotective index, neuroprotective ratio and plasma kynurenic acid concentrations are biological markers to be used to differentiate between normal and depressed persons.

### Example 2: Determination of kynurenine and kynurenic acid using High Performance Liquid Chromatography

1. Introduction:
Combined measurement of kynurenine and kynurenic acid by HPLC with external standardisation. Kynurenine is detected by UV and kynurenic acid by its enhanced fluorescence in a Zn containing elution solvent.
2. Sample preparation:
Samples (serum, whole blood, plasma) need to be deproteinated. Add 20 µl of perchloric acid to 180 µl of sample.
Leave for 10 minutes, then centrifuge for 5 minutes at 14000 g. The 100 µl of the supernatant is transferred to a Gilson injection vial, capped and centrifuged for 5 minutes an 6000 g.
3. Instrumentation: configuration system 2
3.1 Gilson 305 HPLC pump
3.2 Gilson 231 autoinjector:
   check dilutor reservoir: 20% ACN/AD.
   Program file 1.
   ANAL. TIME = 4 minutes
   SAMPLE NUMBER = number of injections
   INJECTION VOLUME = 40 µl
3.3 Fluorescence detector Shimadzu 10A(xl):
   Excitation: 334 nm
   Emission: 388 nm
   SENS: 1
   GAIN: 3
3.4 Gilson 117 detector
   Wavelength: 365 nm
   Sensitivity 1: 0.01
   Sensitivity 2: 0.1
   Min. peak width: 8 sec
3.5 Gilson Unipoint software:
   Method File: KYNS2.GCT.
   Stop file: STOPKA2.GCT
   Analysis File: KAS2.GAN and KYNS2.G

4. Chromatography:
column: Chromolith Performance 4.6 X 100 mm with a Chromolith guard cartridge.
Buffer: 250 mM Zn-acetate in AD (27.4 g in 500 ml). pH is brought to 5.8 with acetic acid and made up to a volume of 455 ml with water in a measuring cylinder. To this 45 ml ACN (for gradient chromatography) is added.
Solvent is degassed by ultrasonification during 20 minutes.
5. Preparation of standards:
5.1 Kynurenine stock standard is prepared by weighing off 20 mg of kynurenine (MW 208.2) and dissolving in water in a 100 ml measuring flask. Standard is stored as 1 ml aliquots in Eppendorf cups at -20°C.
5.2 Kynurenic acid stock standard is prepared by weighing off 20 mg of kynurenic acid (MW 189.2) and dissolving on EtOH with 1 ml of HCl 12 N in a 100 ml measuring flask. Standard is stored at - 20°C.
5.3 Working standard:
   Dilute stock standards till concentrations of 5 µM for kynurenin (500 µl) and 100 nM for kynurenic acid (10.0 µl) in AD (till 100 ml). Stable for one day at room temperature.

6. Reagents:
Perchloric acid (2.4 M): 7 ml of perchloric acid + 13 ml AD.
7. Quality control:
Commercial drug free plasma (TRP, KYN, KA)
8. Procedure:
- Prepare solvents, reagents, thaw Working Standard
   Solution and Control Plasma.
- Stabilize column
- Check plumbery
- Fluorescence detector
- Sample preparation
- adapt Operation File to number of standard injections, samples and controls.
- Program autoinjector - Start run
- System shut-down.

9. Normal values:
9.1 Clin Chem 44:4,858-62 (1992):
KYN: 1.98 (median) range 1.86-2.10 µM n = 72
9.2 J Chrom B, 675 (1996),157-61:

| | | |
|---|---|---|
| KYN: 1.35 | 0.7 - 3.0 µM | n = 35 |
| KA: 23 | 6 - 54 nM | |

9.3 Anal Bioch 172,518-25 (1988):

| | | |
|---|---|---|
| KYN: 2.21 | 1.30 - 3.32 µM | n = 20M+20F |

9.4 Roseneck studie (project 11/01)

| | | |
|---|---|---|
| KYN: 1.66 | 1.04 - 2.28 µM | n = 36 |

### Example 3: Determination of tryptophan and competing amino acids using High Performance Liquid Chromatography

1. Introduction:
   Simultaneous measurement of: Tyrosine (TYR), Valine (VAL), Tryptophan (TRY), Phenylalanine (PHE), Isoleucine (ILE), Leucine (LEU)
   Other amino acids which elute: Taurine (TAU), a-Amino-n-butyric acid (ABA), Ethanolamine, Methionine (MET)
2. Sample preparation:
   25 µl of sample is diluted with 25 µl of Internal Standard solution (IS) in a Gilson Sample vial.
3. Instrumentation:
   3.1 Gilson System 1
   3.2 ASTED:
      Install 20ul injection loop and dialyser block. Use Prime Solution for Dilutor 0 and Dialyser Solution for Dilutor 1. Place Borate Buffer in vial positions A and B of Rack 50. Place OPA derivatisation reagent in position C.
   3.3 Shimadzu RF-A Fluorescence detector:
      Excitation: 330
      Emission: 450
      SENS: 1
      GAIN: 2
   3.4 Gilson Unipoint Software:
      Control method: AZP
      Analysis method: AZP
4. Chromatography:
   Column: Econospher C18, 3 µm, 4.7 X 50 mm
   Solvent A: 57.2 g Na₂HPO₄.12H₂O or 22.6 9 Na₂HPO₄.anh.
      is dissolved in 400 ml of water by stirring and gently heating. The pH is brought to 6.5 with phosphoric acid and made up to a volume of 1840 ml with water in a measuring cylinder. To this is added 160 ml acetonitrile for chromatography.
   Solvent B: 420 water/280 ACN/320 MeOH (all Spectrograde).
   Both solvents are degassed by ultrasonification during 20 minutes.
5. Preparation of standards:
   - Separate amino acid standards are prepared by weighing off 100 mg of each compound and dissolving in water in a 100 ml measuring flask.
   - Working Standard solution: 5 ml of the following separate amino acid standards are dissolved in 250 ml of water in a measuring flask: TYR, VAL, TRY, PHE, ILE, LEU.
   - Check Standard Solution: same as Working Standard Solution but with these additional compounds: TAU, ABA, MET. This solution is not used for standardisation but for checking chromatogram.
   - Both standards are stored as 1 ml aliquots in Eppendorf cups at -20°C. - Working solution's actual concentrations (in microM): see Peak Table
6. Reagents:
   6.1 Prime Solvent (1 week): 8.6 g NaCl is dissolved in 1 I of water. 500 µl of Triton X 100 is added. Mix well and ultrasonicate during 10 minutes.
   6.2 Dialysis solution (1 week): 0.2 M KH2PO4 with NaN3 (a spoon's tip)
   6.3 Internal Standard Stock Solution: 100 mg Norvaline is dissolved in 100 ml of water. 1.5 ml aliquots are stored in Eppendorf cups at -20°C (2 years).
   6.4 Internal Standard (IS): 1 ml of Internal Standard Stock Solution is diluted with 20 ml of 2 g/l Na₂-EDTA (Kestranal 2S) in a scintillation vial (1 month).
   6.5 Borate buffer: 3.1 g of boric acid is dissolved in 400 ml of water, brought to pH 9.5 with NaOH conc. and diluted till 500 ml (1 month).
   6.6 OPA: 500 mg of orthophtalicdialdehyde (OPA) is dissolved by short ultrasonification in 10 ml of methanol spectrograde in a 100 ml Erlenmeyer flask. 90 ml of Borate Buffer and 500 µl of mercaptoethanol are added. This reagent is stable during 1 month but 50 µl of mercaptoethanol is added every 2 days.
7. Quality control:
   A pool of EDTA plasma is stored in Eppendorf tubes at - 20°C.
8. System shut-down:
   - Rinse column with solvent B
   - Prime ASTED with water
9. Procedure:
   - Prepare solvents, reagents, IS, thaw Working Standard Solution and Control Plasma.
   - Stabilize column by running gradient 3 times: first power on pump 1, pump 2, (eventually prime pumps with column disconnected), Qata Master, Dilutors, ASTED, printer, computer screen, computer. Select method AZP2 and run three times (click "Continue"). Check gradient at least once with and without sample preparation.
   - Prepare ASTED for dialyzing (if changing configuration first run FILE 181): place reagents and solvents, Prime. Run File 150 (ELUT. TIME = 50)
   - Fluorescence detector
   - start stabilizing column
   - check plumbery
   - program ASTED for check sample (Working Standard)
   - If the check sample is not OK, try remedying and start again.

In case of doubt, run Control Standard and/or Quality Control Plasma.
- Sample preparation
- adapt Gilson Program according to number of standard injections, samples and controls.
- Program ASTED.
- Start run.
- System shut-down.

### Example 4: Determination of Hydroxy-Anthranilic Acid using High Performance Liquid Chromatography

1. Introduction:
KYN may be detected by UV and HAA by its fluorescence.
2. Sample preparation:
Samples (serum, EDTA whole blood, EDTA plasma, heparinised or citrated plasma) are first centrifuged at 4000 g to remove particulates. They need to be deproteinated.
Add 20 µl of perchloric acid (6.3) to 180 µl of sample.
Leave for 5 minutes, then centrifuge for 5 minutes at 14000 g. Then 100 µl of the supernatant is transferred to a Gilson injection vial, capped and centrifuged for 5 minutes at 6000 g.
3. Instrumentation:
Configuration System 2
3.1 Gilson 305 HPLC pump.
3.2 Gilson 234 autoinjector:
   Check dilutor reservoir: 20% ACN/AD.
   Program file 3.
   ANAL. TIME = 12 minutes,
   sample number = number of injections,
   INJECTION VOLUME = 45 µl.
3.3 Fluorescence detector Shimadzu 10A(xl):
   Excitation: 316 nm,
   Emission: 420 nm,
   SENS: 1,
   GAIN: 3.
3.5 Gilson Unipoint software:
   Method File: HAAS2.GCT,
   Stop file: STOPSYS2.GCT,
   Analysis File: HAAS2.GAN and HAAS2.GAN.

4. Chromatography:
Column: Prevail select C18 3µ 4.6 X 100 mm (Alltech 99302).
Buffer: 20 mM acetic acid (1.2 ml in 900 ml) is brought to pH 5.8 with KOH and made up to a volume of 1000 ml with water in a measuring cylinder. To this is added 20 ml MeOH (for gradient chromatography). The solvent is degassed by ultrasonification for 10 minutes.

5. Preparation of standards:
5.2 Hydroxyanthranilic acid stock standard is prepared by weighing off 20 mg of anthranylic acid (MW) and dissolving with 40 mM acetate citrate pH 4.5 in a 100 ml measuring flask. Standard is stored at -80.
5.3 Working standard: Dilute stock standards till concentration of 100 nM for hydroxyanthranylic acid (10.0 µl) in AD (till 100 ml).

6. Reagents:
6.3 Perchloric acid (2.4 M): 7 ml of perchloric acid + 13 ml AD
6.4 Internal standard working solution: 5X dilution of 6.4 in 6.3)

7. Procedure:
- Prepare solvents, reagents, thaw Working Standard Solution and Control Plasma,
- Stabilize column,
- Check plumbery,
- Fluorescence detector,
- Sample preparation,
- adapt Operation File to number of standard injections, samples and controls,
- Program autoinjector - Start run,
- System shut-down.

8. Normal values:
8.1 J Chrom B, 675 (1996), 157-61: serum

| | | |
|---|---|---|
| KYN: 1.35 | 0.7 - 3.0 µM | n = 35 |
| HAA: 79 | 15 - 209 nM | |

### Example 5: Determination of Elevated 3-Hydroxykynurenine serum levels in Alzheimer's Disease

### Methods

### Patients and control subjects

Serum levels of TRP, KYN, KYNA, and 3-HK in 20 patients with the diagnosis of clinical probable Alzheimer's disease (AD), 20 patients with late-onset major depression (MD), and 20 healthy elderly subjects with subjective memory complaints (SCI) in whom a neurodegenerative disorder had been ruled out by medical examination and neuropsychological testing were investigated.

For subjects' characteristics, see Table 2. The clinical diagnosis of probable AD is made according to the National Institute of Neurological and Communicative Diseases and StrokelAlzheimer's Disease and Related Disorders Association criteria (McKhann,G., Drachman,D., Folstein,M., Katzman,R., Price,D., and Stadlan,E.M. (1984). Clinical diagnosis of Alzheimer's disease: report of the NINCDS-ADRDA Work Group under the auspices of Department of Health and Human Services Task Force on Alzheimer's Disease. Neurology 34, 939-944.). The diagnosis of major depression is made according to the criteria of ICD-10 and DSM-IV (American Psychiatric Association (1994). Diagnostic and Statistical Manual of Mental Disorders, Fourth Edition Text Revision. (Washington, DC: American Psychiatric Association)). Mean duration of illness in the AD group is 2.20 years (0-5 years). All AD patients are free of antidementive treatment, and have no actual treatment with NSAIDs (Non-Steroidal Anti-imflammatory Drugs). One of the AD patients had a previous history of remitting-relapsing major depression, but is admitted to the hospital without any depressive symptoms and free of anti-depressive medication. On admission three of the MD patients are treated with selective serotonin reuptake inhibitors, one with a tricyclic antidepressant and one with a combination of antidepressants; the remaining MD patients are free of antidepressant medication at the time of blood sampling. One SCI subject suffers from a mild depressive episode and is treated with reboxetine, while another SCI subject is sporadically taking NSAIDs due to ankylosing spondylarthritis.

There is a significant between group difference in age (Kruskal-Wallis test X² = 18.223, p <0.001), gender (X² = 6.667, p =0.036), and Mini-Mental State Examination (MMSE) score (X² = 31.944, p <0.001, see Table 2). Patients with AD are older than patients with MD (Mann-Whitney test Z = -2.654, p =0.008) and SCI subjects (Z = -3.998, p <0.001), and MD patients are older than the SCI subjects (Z = -2.072, p =0.038).

Serum samples are collected in vacucontainers without further additives. After 0.5 hours of coagulation, samples are centrifuged and the supernatant is aliquoted into Eppendorf cups (Eppendorf, Hamburg, Germany) and immediately frozen at -80°C.

### Laboratory analyses

We established a gradient high-performance liquid chromatographic (HPLC) method with ultraviolet (UV) and fluorescence detection.

### Chemicals

Reagents for solid phase extraction and chromatography are purchased from Merck (Darmstadt, Germany) in gradient grade purity for liquid chromatography. Ultra pure water is produced by a Millipore Milli-Q system (Millipore, Milford, MS). Phosphate buffered saline (PBS) capsules (Sigma, St. Louis, MO) are used to generate a 0.05 M PBS. Tryptophan, kynurenine, kynurenic acid, and 3-hydroxykynurenine are purchased in high purity from Sigma (St. Louis, MO).

Calibrators and controls are established by adding defined concentrations of the analytes to a 0.05 M PBS solution. The following concentrations are used for five-point calibration: TRP: 0.313, 0.625, 1.25, 2.5, 5.0, 10.0, 20.0 µg/ml; KYN: 12.5, 25.0, 50.0, 100, 200, 400, 800 ng/ml; KYNA: 4.688, 9.375, 18.75, 37.5, 75.0, 150, 300 ng/ml; 3-HK: 1.563, 3.125, 6.25, 12.5, 25.0, 50.0, 100 ng/ml.

### Sample extraction

Analytes are extracted from samples and calibrators/controls using Waters Oasis MCX 1 cc (30 mg) extraction cartridges (Waters, Milford, MS) as follows (all extractions are conducted with a manual vacuum-manifold system): (1) the cartridge is preconditioned by rinsing with 1 ml of methanol followed by 1 ml water; (2) 1 ml sample and 100 µl 1M H₃PO₄ are applied to the cartridge and pulled through under a light vacuum (2 minutes); (3) the cartridge is washed with 1 ml of 0.1 M HCl followed by 1 ml 100% methanol; and finally, (4) the analytes are eluted by rinsing the cartridge with 1.5 ml of acetonitrile containing 6% NaOH. The eluent is then evaporated under nitrogen to dryness and reconstituted with 150 µl 0.1 M PBS. The reconstituted sample/calibrator/control is then transferred to a microinjection vial (Waters).

### HPLC Equipment and chromatographic conditions

Analyses are carried out on a Waters 2695 chromatograph connected to a Waters Model 2487 dual-λ UV detector and a 2475 fluorescence detector.

For the determination of KYN, KYNA, and 3-HK, 100 µl of the samples are loaded onto an 250 mm x 4 mm Supersphere 60 RP-select B, C8 column (Merck, Darmstadt, Germany). Due to the relatively higher concentration, a second injection with a volume of 10 µl is performed for the determination of TRP. In order to ensure optimal peak resolution in the chromatograms, and hence efficient separation of the analytes in a reasonably short time (30 minutes), elution is carried out in the gradient mode using a mobile phase consisting of a mixture of 0.050 M sodium acetate (solvent A: pH 4.80; solvent B: pH 3.65), acetonitrile (solvent C), and methanol (solvent D) at distinct proportions (see Table 3).

Flow rate is set at 0.80 ml/minute, column temperature is set at 35.0 °C, while the samples are cooled at 4.0°C. TRP is measured by fluorescence detection (λex: 300 nm; λem: 350 nm), KYN (365 nm), KYNA (330 nm), and 3-HK (365 nm) are measured by UV detection. Approximate run time after injection until detection of the compounds is about 20.4 minutes for TRP, 13.4 minutes for KYN, 22.5 minutes for KYNA, and 7.0 minutes for 3-HK.

Data are processed using EMPOWER for Windows 2000 software (Waters). The concentrations are established through comparison of peak heights of the single analytes with the peak heights of the respective calibration curves.

### Statistics

Statistical analysis is performed with SPSS (version 12.0.1; SPSS, Chicago, Illinois) with nonparametric procedures (Kruskal-Wallis-Test - Mann-Whitney-Test - Spearman-Rank correlation). Level of significance is set at p <0.050. To control significant between group effects for differences in age, a linear model with diagnosis as independent factor and age as covariate is used. Because the outcome variables are not normally distributed by visual inspection of the regression residuals and Kolmogorov Smirnov tests (p < 0.05), bootstrapping applied to a multiple regression model with diagnosis and age as independent predictor variables for a distribution free significance test is used. Specifically, the multiple regression model for each pair of diagnoses on the basis of 999 samples is iteratively computed. To assess whether a marker showing significant differences between the AD and the comparison groups might also be useful as a potential diagnostic test, sensitivity of the marker when specificity is set at > 80% and specificity when sensitivity is set at > 80% using ROC analysis is determined. The level of 80% is chosen based on the consensus criteria for a clinically useful biomarker in AD (The Ronald and Nancy Reagan Research Institute of the Alzheimer's Association and the National Institute on Aging Working Group (1998). Consensus report of the Working Group on: "Molecular and Biochemical Markers of Alzheimer's Disease". The Ronald and Nancy Reagan Research Institute of the Alzheimer's Association and the National Institute on Aging Working Group. Neurobiol. Aging 19, 109-116.).

### Results

Mean serum levels of tryptophan (TRP), kynurenine (KYN), kynurenic acid (KYNA), and 3-hydroxykynurenine (3-HK) in patients with Alzheimer's disease (AD), patients with major depression (MD), and healthy persons with subjective cognitive impairment (SCI) are given in Table 4. There is a non-significant difference of TRP levels between the three groups (Kruskal-Wallis: X² = 5.507; p =0.064) with AD patients showing lower TRP levels than healthy control persons (Mann-Whitney Z = - 2.288; p =0.022). The serum levels of KYN (X² = 1.536; p = 0.464) and KYNA (X² = 0.033; p = 0.984) are not different between the groups. In contrast, 3-HK levels are significantly different between the three groups (X² = 20.281; p<.0001), with higher serum 3-HK levels in AD patients compared to the patients with major depression (Z = -3.571; p <0.001) and SCI controls (Z = -4.139; p <0.0001). In contrast, serum 3-HK levels did not differ between patients with MD and SCI controls (Z = -.541; p =0.602). Since availability of the essential amino acid TRP is a limiting factor for the production of its metabolites, the ratio between 3-HK and TRP levels is also calculated. Again, there is a highly significant difference between the groups (X² = 21.911; p <0.0001) with a much higher mean ratio (3-HK/TRP) in AD patients than in MD patients (Z = -3.517; p <0.001) and healthy controls (Z = -4.436; p <0.0001), but with no difference between the two comparison groups (Z = -0.757; p = 0.449). Accordingly, the ratio between neurotoxic 3-HK and neuroprotective KYNA is significantly increased in AD patients (X² = 18.016, p =0.0009; AD vs. MD: Z = - 3.219, p = 0.001; AD vs. SCI: Z = -4.003, p = 0.00002; MD vs. SCI: Z = -0.649, p = 0.529).

When the significant between group differences in 3-HK and 3-HK/TRP are controlled for age using bootstrapping for determination of the sampling distribution, 3-HK levels are significantly different between AD patients and SCI controls (partial correlation coefficient -0.42, p <0.05) and between AD and MD patients (partial correlation coefficient -0.35, p <0.05). The ratio of 3-HK to TRP is significantly different between AD patients and SCI controls (partial correlation coefficient -0.47, p <0.05), but not between AD and MD patients (partial correlation coefficient -0.2).

Using ROC analysis of the AD group compared with the combined MD and SCI group, for 3-HK sensitivity is 75% when specificity is set at 85%, and specificity is 70% when sensitivity is set at 90%. For the ratio of 3-HK to TRP sensitivity is 80% when specificity is set at 82.5%, and specificity is 77.5% when sensitivity is set at 85%.

### Tables

**Table 1**

| Group | Gender | Age | TYR | VAL | TRP | PHE | ILE | LEU | Kyn | KA | TRPi | KYN/TR P | KAKYN | PROi |
|---|---|---|---|---|---|---|---|---|---|---|---|---|---|---|
| Disease | M | 68 | 87.14 | 263.7 | 55.31 | 96.47 | 66.66 | 129.95 | 2.99 | 31.84 | 8.59 | 0.0540 | 10.67 | 339.61 |
| Disease | M | 44 | 79.93 | 376.89 | 88.94 | 87.77 | 137.2 | 226.64 | 2.51 | 29.33 | 9.79 | 0.0282 | 11.71 | 343.30 |
| Disease | F | 35 | 92.87 | 260.05 | 62.23 | 82.86 | 96.06 | 156.65 | 2.06 | 24.96 | 9.04 | 0.0331 | 12.11 | 302.29 |
| Disease | M | 31 | 63.29 | 230.9 | 62.43 | 68.38 | 76.53 | 125.46 | 1.85 | 17.87 | 11.06 | 0.0296 | 9.66 | 172.63 |
| Disease | F | 52 | 63.12 | 246.49 | 84.73 | 81.67 | 82.9 | 141.06 | 1.49 | 14.34 | 13.77 | 0.0176 | 9.62 | 137.98 |
| Disease | F | 68 | 105.1 | 376.3 | 101.54 | 110.33 | 140.93 | 198.4 | 1.90 | 26.81 | 10.91 | 0.0188 | 14.08 | 377.39 |
| Disease | M | 20 | 68.26 | 300.6 | 89.45 | 110.23 | 96.08 | 192.06 | 1.80 | 33.71 | 11.66 | 0.0201 | 18.70 | 630.54 |
| Disease | F | 34 | 57.66 | 210.25 | 47.97 | 69.85 | 89.7 | 125 | 1.63 | 11.60 | 8.68 | 0.0339 | 7.13 | 82.70 |
| Disease | M | 61 | 57.29 | 241.99 | 58 | 65.2 | 78.73 | 117 | 1.33 | 13.79 | 10.35 | 0.0229 | 10.38 | 143.18 |
| Disease | F | 67 | 52.67 | 251.93 | 59.7 | 76.58 | 74.52 | 128.63 | 1.93 | 28.16 | 10.22 | 0.0324 | 14.56 | 409.90 |
| Disease | F | 30 | 66.08 | 245.14 | 75.71 | 94.63 | 83.71 | 154.31 | 1.43 | 16.71 | 11.76 | 0.0189 | 11.68 | 195.18 |
| Disease | M | 40 | 48.34 | 193.21 | 53.76 | 61.03 | 55.14 | 94.44 | 1.52 | 24.85 | 11.89 | 0.0283 | 16.35 | 406.26 |
| Disease | F | 23 | 62 | 317 | 51.3 | 80 | 80 | 191 | 1.44 | 21.87 | 7.03 | 0.0281 | 15.19 | 332.15 |
| Disease | M | 46 | 90.82 | 318.26 | 104.36 | 102.49 | 106.22 | 188.57 | 2.46 | 21.47 | 12.94 | 0.0236 | 8.73 | 187.38 |
| Disease | M | 33 | 72.31 | 418.11 | 86.75 | 104.43 | 112.24 | 195.43 | 3.94 | 56.29 | 9.61 | 0.0454 | 14.29 | 804.20 |
| Disease | F | 51 | 52.06 | 215.4 | 54.45 | 74.58 | 92.56 | 134.33 | 1.81 | 35.54 | 9.57 | 0.0332 | 19.64 | 697.84 |
| Disease | F | 50 | 106.3 | 357.06 | 84.05 | 95.28 | 127.88 | 204.96 | 1.82 | 35.21 | 9.43 | 0.0217 | 19.35 | 681.18 |
| Disease | M | 20 | 72.26 | 343.18 | 85.84 | 96.24 | 128.5 | 220.42 | 1.91 | 25.86 | 9.97 | 0.0222 | 13.54 | 350.12 |
| Disease | M | 37 | 53.39 | 257.99 | 64.99 | 64.92 | 86.14 | 143.17 | 2.91 | 19.80 | 10.73 | 0.0448 | 6.80 | 134.58 |
| Disease | F | 52 | 80.22 | 267.89 | 77.55 | 83.11 | 90.69 | 169.15 | 1.85 | 14.81 | 11.22 | 0.0239 | 8.01 | 118.56 |
| Disease | F | 61 | 78.16 | 237.18 | 49.78 | 103.12 | 95 | 135.81 | 1.98 | 25.47 | 7.67 | 0.0398 | 12.86 | 327.64 |
| Disease | F | 41 | 71.59 | 309.87 | 54.17 | 114.52 | 97.29 | 216.86 | 1.06 | 27.26 | 6.69 | 0.0196 | 25.72 | 701.04 |
| Disease | M | 39 | 79.65 | 278.68 | 66.04 | 89.76 | 98.05 | 183.32 | 1.92 | 28.56 | 9.05 | 0.0291 | 14.88 | 424.83 |
| Disease | M | 45 | 54.56 | 295.44 | 42.61 | 78.96 | 123.37 | 208.21 | 1.20 | 25.59 | 5.60 | 0.0282 | 21.33 | 545.71 |
| Disease | M | 36 | 59.25 | 276.07 | 61.44 | 79.84 | 82.34 | 155.45 | 1.46 | 26.28 | 9.41 | 0.0238 | 18.00 | 473.04 |
| Disease | F | 38 | 96.4 | 306 | 79.9 | 94.2 | 93.2 | 166 | 1.42 | 19.00 | 10.57 | 0.0178 | 13.38 | 254.23 |
| Disease | F | 35 | 53.12 | 188.28 | 46.91 | 56.48 | 57.41 | 101.99 | 0.75 | 15.94 | 10.26 | 0.0160 | 21.25 | 338.78 |
| Disease | M | 20 | 71.83 | 276.7 | 88.19 | 93.49 | 78.02 | 178.17 | 1.77 | 36.26 | 12.63 | 0.0201 | 20.49 | 742.82 |
| Disease | M | 32 | 82.7 | 306.59 | 57.24 | 86.81 | 101.51 | 198.25 | 1.72 | 24.70 | 7.38 | 0.0300 | 14.36 | 354.70 |
| Disease | F | 21 | 62.45 | 232.87 | 69.74 | 88.74 | 75.78 | 154.07 | 2.14 | 20.09 | 11.36 | 0.0307 | 9.39 | 188.60 |
| Disease | M | 63 | 65.45 | 344.26 | 57.84 | 92.89 | 127.58 | 223.23 | 2.25 | 31.35 | 6.78 | 0.0389 | 13.93 | 436.81 |
| Disease | M | 67 | 66.36 | 282.86 | 59.19 | 82.04 | 72.61 | 152.41 | 2.06 | 26.97 | 9.02 | 0.0348 | 13.09 | 353.10 |
| Disease | F | 40 | 63.58 | 244.54 | 70.29 | 72.7 | 72.23 | 141.26 | 1.01 | 20.36 | 11.83 | 0.0144 | 20.16 | 410.43 |
| Disease | M | 60 | 69.79 | 316.34 | 35.02 | 85 | 106.15 | 187.38 | 1.22 | 24.47 | 4.58 | 0.0348 | 20.06 | 490.80 |
| Disease | F | 42 | 64.29 | 243.52 | 60.87 | 69.42 | 72.54 | 142.77 | 1.42 | 23.05 | 10.27 | 0.0233 | 16.23 | 374.16 |
| Disease | M | 41 | 60.03 | 225.76 | 48.89 | 66.74 | 69.3 | 123.49 | 2.01 | 22.78 | 8.97 | 0.0411 | 11.33 | 258.17 |
| Disease | M | 42 | 66.65 | 270.91 | 72.22 | 74.28 | 92.07 | 169.43 | 1.79 | 31.10 | 10.73 | 0.0248 | 17.37 | 540.34 |
| Disease | F | 60 | 64.52 | 227.57 | 50.73 | 60.26 | 71.34 | 113.87 | 1.79 | 21.82 | 9.44 | 0.0353 | 12.19 | 265.98 |
| Disease | M | 52 | 47.94 | 207.97 | 52.33 | 65.94 | 114.84 | 57.91 | 1.84 | 19.90 | 10.58 | 0.0352 | 10.82 | 215.22 |
| Disease | M | 49 | 64.68 | 283.25 | 62.09 | 59.75 | 79.29 | 148.15 | 1.39 | 17.80 | 9.78 | 0.0224 | 12.81 | 227.94 |
| Disease | M | 66 | 50.5 | 207.19 | 38.14 | 62.38 | 65.21 | 117.37 | 1.35 | 10.78 | 7.59 | 0.0354 | 7.99 | 86.08 |
| Disease | F | 48 | 70.79 | 313.1 | 74.21 | 67.66 | 85.85 | 154.31 | 1.71 | 24.61 | 10.73 | 0.0230 | 14.39 | 354.18 |
| Disease | F | 43 | 70.55 | 210.41 | 70.69 | 78.76 | 65.47 | 123.65 | 1.63 | 26.04 | 12.88 | 0.0231 | 15.98 | 416.00 |
| Disease | M | 43 | 42.96 | 204.91 | 55.37 | 55.74 | 76.35 | 125.37 | 1.88 | 13.61 | 10.96 | 0.0340 | 7.24 | 98.53 |
| Disease | F | 43 | 84.69 | 212.39 | 57.9 | 68.17 | 55.94 | 117.44 | 1.41 | 23.44 | 10.75 | 0.0244 | 16.62 | 389.67 |
| Disease | F | 24 | 56.28 | 264.5 | 66.27 | 73.73 | 78.58 | 167.06 | 1.42 | 15.56 | 10.35 | 0.0214 | 10.96 | 170.50 |
| Disease | F | 68 | 73.73 | 257.95 | 69.25 | 78.04 | 82.88 | 140.21 | 2.02 | 34.10 | 10.94 | 0.0292 | 16.88 | 575.65 |
| average | | 44.277 | 68.587 | 270.584 | 65.242 | 80.967 | 89.204 | 156.172 | 1.791 | 24.291 | 9.894 | 0.028 | 14.082 | 358.765 |
| average dev | | 11.424 | 11.28 | 41.924 | 12.803 | 12.545 | 16.759 | 30.543 | 0.3677 | 5.8735 | 1.4505 | 0.0068 | 3.4879 | 142.69 |
| Normal | M | 26 | 85.92 | 280.85 | 66.2 | 80.74 | 81.71 | 155.62 | 2.60 | 35.93 | 9.67 | 0.0393 | 13.81 | 496.23 |
| Normal | M | 33 | 63.48 | 256.2 | 71.5 | 84.52 | 82.38 | 156.44 | 1.23 | 16.66 | 11.12 | 0.0171 | 13.59 | 226.35 |
| Normal | F | 38 | 50.86 | 257.27 | 74.66 | 64.11 | 76.45 | 135.14 | 2.10 | 55.74 | 12.79 | 0.0281 | 26.54 | 1479.50 |
| Normal | F | 25 | 50.52 | 226.25 | 47.26 | 64.66 | 60.52 | 114.16 | 1.74 | 32.69 | 9.16 | 0.0368 | 18.79 | 614.16 |
| Normal | F | 26 | 57.18 | 272.04 | 69.04 | 79.9 | 78.33 | 141.88 | 1.74 | 30.81 | 10.97 | 0.0252 | 17.71 | 545.55 |
| Normal | M | 31 | 77.22 | 295.32 | 64.78 | 86.19 | 95.89 | 170.89 | 1.66 | 53.85 | 8.93 | 0.0256 | 32.44 | 1746.88 |
| Normal | F | 23 | 75.91 | 235.17 | 65.24 | 60.12 | 65.54 | 122.87 | 1.41 | 36.05 | 11.66 | 0.0216 | 25.57 | 921.70 |
| Normal | F | 24 | 75.28 | 306.84 | 89.74 | 85.65 | 104.99 | 188.73 | 2.92 | 48.31 | 11.78 | 0.0325 | 16.54 | 799.27 |
| Normal | F | 43 | 81.76 | 364.43 | 82.47 | 99.37 | 132.87 | 217.79 | 2.64 | 36.53 | 9.20 | 0.0320 | 13.84 | 505.47 |
| Normal | F | 22 | 66.59 | 232.12 | 59.67 | 72.02 | 69.32 | 126.1 | 1.70 | 20.62 | 10.54 | 0.0285 | 12.13 | 250.11 |
| Normal | F | 39 | 98.54 | 389.11 | 88.22 | 85.16 | 151.75 | 243.89 | 1.46 | 26.57 | 9.11 | 0.0165 | 18.20 | 483.54 |
| Normal | M | 32 | 73.14 | 223.15 | 75.48 | 80.12 | 73.22 | 129.57 | 1.58 | 22.35 | 13.03 | 0.0209 | 14.15 | 316.15 |
| Normal | F | 38 | 42.31 | 174.21 | 53 | 69.31 | 54.98 | 98.29 | 1.43 | 22.51 | 12.07 | 0.0270 | 15.74 | 354.29 |
| Normal | F | 14 | 88.22 | 238.34 | 91.17 | 87.04 | 66.5 | 117.3 | 2.49 | 61.06 | 15.26 | 0.0273 | 24.52 | 1497.32 |
| Normal | M | 60 | 64.28 | 289.74 | 63.71 | 80.74 | 85.93 | 165.58 | 1.84 | 23.63 | 9.28 | 0.0289 | 12.84 | 303.47 |
| Normal | M | 35 | 45.61 | 217.82 | 48.39 | 54.85 | 46.58 | 94.08 | 1.51 | 25.13 | 10.54 | 0.0312 | 16.64 | 418.22 |
| Normal | M | 29 | 61.03 | 231.44 | 63.57 | 67.57 | 69.5 | 104.66 | 1.44 | 30.70 | 11.90 | 0.0227 | 21.32 | 654.51 |
| Normal | M | 49 | 77.08 | 208.37 | 73.35 | 68.73 | 65.18 | 121.83 | 2.05 | 65.47 | 13.55 | 0.0279 | 31.94 | 2090.89 |
| Normal | M | 18 | 70.9 | 308.78 | 75.86 | 78.37 | 81.05 | 172.04 | 1.48 | 30.15 | 10.67 | 0.0195 | 20.37 | 614.20 |
| Normal | F | 22 | 49.46 | 206.08 | 61.21 | 61.94 | 58.98 | 116.05 | 1.42 | 33.82 | 12.43 | 0.0232 | 23.82 | 805.49 |
| Normal | M | 27 | 125.39 | 512.88 | 136.14 | 128.46 | 161.38 | 272.7 | 2.34 | 56.99 | 11.34 | 0.0172 | 24.35 | 1387.97 |
| Normal | F | 21 | 79.65 | 340.52 | 83 | 76.15 | 99.53 | 185.28 | 1.67 | 41.63 | 10.63 | 0.0201 | 24.93 | 1037.76 |
| Normal | M | 34 | 74.22 | 289.78 | 84.62 | 82.71 | 102.22 | 175.94 | 1.22 | 29.44 | 11.67 | 0.0144 | 24.13 | 710.42 |
| Normal | M | 24 | 52.4 | 252 | 50 | 57.3 | 77.2 | 124 | 2.14 | 28.86 | 8.88 | 0.0429 | 13.46 | 388.33 |
| Normal | F | 24 | 70.59 | 333.52 | 57.99 | 70.88 | 94.83 | 155.29 | 2.19 | 35.85 | 8.00 | 0.0378 | 16.37 | 586.86 |
| Normal | M | 32 | 56.72 | 252.22 | 59.14 | 62.77 | 71.3 | 122.58 | 1.20 | 12.13 | 10.46 | 0.0203 | 10.11 | 122.61 |
| Normal | F | 22 | 103.82 | 319.45 | 80.37 | 86.42 | 125.75 | 203.7 | 0.98 | 25.15 | 9.58 | 0.0122 | 25.66 | 645.43 |
| Normal | M | 31 | 72.57 | 210.37 | 53.19 | 64.8 | 58.92 | 129.02 | 1.96 | 25.65 | 9.93 | 0.0368 | 13.09 | 335.67 |
| Normal | F | 40 | 47.52 | 236.7 | 58.23 | 65.74 | 115.79 | 72.11 | 1.73 | 43.77 | 10.83 | 0.0297 | 25.30 | 1107.41 |
| Normal | M | 23 | 50.62 | 230.92 | 57.6 | 66.21 | 119.85 | 81.02 | 1.75 | 32.98 | 10.50 | 0.0304 | 18.85 | 621.53 |
| Normal | M | 52 | 56.79 | 252.36 | 59.25 | 67.99 | 74.41 | 141.64 | 1.40 | 31.97 | 9.99 | 0.0236 | 22.84 | 730.06 |
| Normal | M | 24 | 54.4 | 250.4 | 75.91 | 69.38 | 75.96 | 139.54 | 2.14 | 30.91 | 12.87 | 0.0282 | 14.44 | 446.46 |
| Normal | M | 28 | 68.96 | 261.15 | 79.83 | 91.84 | 86.16 | 148.51 | 1.83 | 37.31 | 12.16 | 0.0229 | 20.39 | 760.68 |
| Normal | M | 33 | 62.44 | 254.3 | 65.66 | 79.68 | 92.25 | 148.38 | 1.87 | 39.17 | 10.31 | 0.0285 | 20.95 | 820.48 |
| Normal | F | 26 | 81.28 | 299.3 | 70.25 | 79.27 | 100.58 | 152.51 | 2.00 | 38.01 | 9.85 | 0.0285 | 19.00 | 722.27 |
| Normal | F | 21 | 73.79 | 387.97 | 71.24 | 88.14 | 131.4 | 197.62 | 1.79 | 36.61 | 8.11 | 0.0251 | 20.45 | 748.77 |
| Normal | M | 27 | 70.53 | 289.96 | 47.2 | 94.29 | 92.02 | 177.07 | 1.69 | 46.32 | 6.52 | 0.0358 | 27.41 | 1269.55 |
| Normal | F | 43 | 75.06 | 273.9 | 65.31 | 79.62 | 94.29 | 174.42 | 1.71 | 48.33 | 9.37 | 0.0262 | 28.26 | 1365.96 |
| Normal | F | 26 | 73.25 | 267.69 | 66.38 | 85.83 | 73.75 | 162.15 | 2.11 | 37.49 | 10.02 | 0.0318 | 17.77 | 666.11 |
| Normal | F | 13 | 67.48 | 228.24 | 89.84 | 75.04 | 60.82 | 117.82 | 2.87 | 55.93 | 16.35 | 0.0319 | 19.49 | 1089.95 |
| Normal | M | 45 | 73.78 | 256.3 | 59.38 | 74.66 | 97.42 | 152.32 | 1.31 | 24.92 | 9.07 | 0.0221 | 19.02 | 474.05 |
| Normal | F | 40 | 69.2 | 308.36 | 71.35 | 77.85 | 94.6 | 173.05 | 1.82 | 32.54 | 9.87 | 0.0255 | 17.88 | 581.79 |
| Normal | F | 48 | 47.69 | 230.78 | 74.85 | 81.22 | 68.3 | 125.92 | 1.64 | 35.70 | 13.51 | 0.0219 | 21.77 | 777.13 |
| Normal | F | 33 | 76.68 | 395.14 | 78.84 | 105.78 | 148.92 | 245.64 | 2.15 | 46.40 | 8.11 | 0.0273 | 21.58 | 1001.38 |
| Normal | M | 41 | 72.17 | 241.48 | 60.83 | 69.24 | 71.42 | 117.68 | 2.07 | 36.98 | 10.63 | 0.0340 | 17.89 | 661.67 |
| Normal | F | 18 | 88.52 | 295.21 | 74.86 | 115.75 | 107.42 | 176.77 | 2.29 | 53.20 | 9.55 | 0.0306 | 23.23 | 1235.91 |
| Normal | M | 21 | 87.65 | 342.55 | 79.53 | 106.66 | 116.75 | 222.82 | 1.62 | 37.09 | 9.07 | 0.0204 | 22.90 | 849.18 |
| Normal | F | 26 | 70.02 | 260.83 | 62.68 | 80.95 | 81.65 | 163.44 | 1.42 | 18.28 | 9.54 | 0.0227 | 12.87 | 235.32 |
| Normal | F | 19 | 65.12 | 342.15 | 72.42 | 89.35 | 104.28 | 159.65 | 2.12 | 36.98 | 9.52 | 0.0293 | 17.44 | 645.06 |
| Normal | F | 37 | 48.27 | 208.26 | 62.53 | 67.99 | 62.8 | 127.45 | 1.79 | 21.62 | 12.15 | 0.0286 | 12.08 | 261.13 |
| Normal | F | 50 | 109.86 | 365.58 | 112.91 | 107.94 | 98.64 | 202.86 | 1.98 | 31.98 | 12.76 | 0.0175 | 16.18 | 517.34 |
| Normal | F | 26 | 92 | 265.49 | 75.53 | 79.26 | 85.52 | 154.04 | 2.34 | 52.98 | 11.17 | 0.0309 | 22.67 | 1201.25 |
| Normal | F | 31 | 42.1 | 206 | 70.4 | 59.1 | 59.1 | 104 | 1.44 | 23.91 | 14.97 | 0.0205 | 16.58 | 396.43 |
| Normal | F | 18 | 71.59 | 259.86 | 61.03 | 67.87 | 82.68 | 122.82 | 1.62 | 29.86 | 10.09 | 0.0265 | 18.48 | 551.71 |
| Normal | F | 37 | 82.3 | 354.52 | 91.04 | 91.48 | 124.62 | 196.72 | 1.95 | 33.09 | 10.72 | 0.0214 | 17.00 | 562.67 |
| Normal | F | 49 | 78.7 | 282.09 | 79.83 | 76.4 | 82.1 | 156.04 | 4.06 | 69.31 | 11.82 | 0.0508 | 17.09 | 1184.49 |
| Normal | F | 22 | 105.24 | 350.8 | 89.12 | 89.9 | 129.96 | 216.99 | 1.58 | 30.51 | 9.98 | 0.0177 | 19.31 | 589.14 |
| Normal | M | 27 | 78.65 | 297.28 | 80.17 | 76.28 | 94.43 | 147.39 | 2.40 | 40.40 | 11.55 | 0.0299 | 16.85 | 680.78 |
| Normal | M | 34 | 89.01 | 336.88 | 87.05 | 106.2 | 128.22 | 229.63 | 2.01 | 24.39 | 9.78 | 0.0231 | 12.16 | 296.49 |
| Normal | M | 31 | 76.95 | 272.3 | 97.91 | 86.12 | 96.04 | 169.29 | 2.05 | 41.36 | 13.97 | 0.0209 | 20.18 | 834.46 |
| Normal | M | 26 | 98.99 | 356.51 | 83.67 | 90.85 | 139.19 | 217.67 | 1.56 | 37.37 | 9.26 | 0.0186 | 23.96 | 895.20 |
| Normal | F | 32 | 66.15 | 298.79 | 85.58 | 66.99 | 100.93 | 165.82 | 1.73 | 33.47 | 12.25 | 0.0202 | 19.35 | 647.54 |
| Normal | M | 37 | 63.65 | 247.58 | 80.1 | 82.73 | 64.41 | 134.1 | 1.66 | 30.32 | 13.52 | 0.0207 | 18.27 | 553.80 |
| Normal | F | 26 | 74.7 | 274.84 | 73.85 | 95.5 | 98.19 | 154.64 | 1.95 | 54.55 | 10.58 | 0.0264 | 27.97 | 1526.00 |
| Normal | M | 43 | 63.45 | 242.48 | 63.4 | 63.36 | 75.36 | 126.95 | 1.96 | 39.45 | 11.09 | 0.0310 | 20.08 | 792.06 |
| Normal | M | 39 | 48.92 | 246.31 | 68.5 | 68.91 | 67.12 | 130.77 | 1.88 | 30.30 | 12.19 | 0.0274 | 16.12 | 488.35 |
| Normal | M | 31 | 102.3 | 337.23 | 79.85 | 88.71 | 109.58 | 184.2 | 2.15 | 23.19 | 9.71 | 0.0269 | 10.79 | 250.13 |
| Normal | M | 29 | 65.05 | 294.12 | 78.39 | 80.25 | 87.29 | 159.73 | 1.88 | 61.03 | 11.42 | 0.0240 | 32.46 | 1981.20 |
| Normal | F | 46 | 86.14 | 291.2 | 56.99 | 78.41 | 84.89 | 145.01 | 2.23 | 59.94 | 8.31 | 0.0391 | 26.88 | 1611.12 |
| Normal | M | 19 | 65.77 | 316.33 | 80.69 | 72.82 | 115.68 | 175.47 | 2.55 | 33.29 | 10.82 | 0.0316 | 13.05 | 434.60 |
| Normal | F | 23 | 67.44 | 286.77 | 66.49 | 61.21 | 69.58 | 126.92 | 1.65 | 22.22 | 10.87 | 0.0248 | 13.47 | 299.23 |
| Normal | F | 19 | 57.39 | 248.82 | 55.97 | 70.29 | 63.96 | 135.13 | 2.38 | 68.78 | 9.72 | 0.0425 | 28.90 | 1987.68 |
| Normal | F | 34 | 49.96 | 171.17 | 51.75 | 75.47 | 52.99 | 97.59 | 1.52 | 27.51 | 11.57 | 0.0294 | 18.10 | 497.89 |
| Normal | M | 26 | 86.4 | 188 | 52.7 | 74.8 | 54.1 | 117 | 1.69 | 34.72 | 10.13 | 0.0321 | 20.54 | 713.30 |
| Normal | F | 33 | 46.46 | 203.96 | 59.74 | 66.81 | 62.2 | 108.33 | 1.27 | 30.27 | 12.25 | 0.0213 | 23.83 | 721.47 |
| Normal | F | 30 | 48.56 | 186.41 | 51.33 | 50.63 | 48.96 | 78.35 | 2.17 | 32.99 | 12.43 | 0.0423 | 15.21 | 501.95 |
| Normal | M | 28 | 62.42 | 189.75 | 60.42 | 80.6 | 59.2 | 98.86 | 1.73 | 58.87 | 12.31 | 0.0286 | 34.03 | 2003.28 |
| Normal | F | 32 | 54.7 | 266.7 | 65.5 | 64.5 | 83.1 | 138.8 | 2.03 | 26.47 | 10.77 | 0.0310 | 13.04 | 345.15 |
| Normal | F | 23 | 43.1 | 220.9 | 56.9 | 89.9 | 60.2 | 114.9 | 1.92 | 83.79 | 10.76 | 0.0337 | 43.64 | 3656.65 |
| Normal | M | 31 | 51.0 | 257.1 | 58.2 | 75.9 | 78.2 | 139.1 | 1.54 | 18.87 | 9.69 | 0.0264 | 12.25 | 231.22 |
| Normal | M | 47 | 71.14 | 317.02 | 75.23 | 85.47 | 95 | 182.33 | 1.94 | 48.08 | 10.02 | 0.0258 | 24.78 | 1191.59 |
| Normal | M | 33 | 61.89 | 334.76 | 70.1 | 66.31 | 98.9 | 174.95 | 1.94 | 30.19 | 9.51 | 0.0277 | 15.56 | 469.81 |
| Normal | M | 67 | 73.12 | 287.99 | 87.47 | 82.46 | 98.67 | 159.65 | 1.37 | 20.10 | 12.46 | 0.0157 | 14.67 | 294.90 |
| Normal | M | 21 | 69.26 | 294.2 | 77.84 | 67.53 | 90.06 | 161.12 | 1.82 | 35.00 | 11.41 | 0.0234 | 19.23 | 673.08 |
| Normal | M | 24 | 84.05 | 275.82 | 73.74 | 71.44 | 84.53 | 155.9 | 2.22 | 26.46 | 10.98 | 0.0301 | 11.92 | 315.37 |
| Normal | M | 20 | 69.79 | 359.13 | 79.53 | 105.21 | 111.28 | 203.68 | 1.90 | 33.78 | 9.37 | 0.0239 | 17.78 | 600.57 |
| Normal | F | 53 | 72.71 | 221.39 | 69.4 | 101.25 | 66.02 | 129.85 | 2.16 | 22.44 | 11.74 | 0.0311 | 10.40 | 233.49 |
| Normal | M | 34 | 76.2 | 298.56 | 71.03 | 85.26 | 90.65 | 155.15 | 1.91 | 34.31 | 10.06 | 0.0269 | 17.96 | 616.32 |
| Normal | M | 20 | 79.68 | 306.32 | 73.96 | 92.73 | 87.98 | 170.59 | 1.91 | 26.98 | 10.03 | 0.0258 | 14.13 | 381.11 |
| Normal | F | 20 | 55.33 | 212.5 | 60.85 | 67.5 | 56.86 | 131.09 | 1.63 | 25.51 | 11.63 | 0.0268 | 15.65 | 399.24 |
| Normal | F | 41 | 52.09 | 185.33 | 72.79 | 67.14 | 55.3 | 87.19 | 1.57 | 27.74 | 16.28 | 0.0216 | 17.67 | 490.13 |
| Normal | M | 69 | 47.92 | 220.64 | 71.21 | 70.24 | 65.09 | 111.4 | 2.30 | 44.41 | 13.82 | 0.0323 | 19.30 | 857.32 |
| Normal | M | 42 | 50.25 | 200.96 | 58.8 | 83.13 | 56.8 | 108.07 | 1.23 | 18.01 | 11.78 | 0.0208 | 14.69 | 264.54 |
| Normal | M | 35 | 63.85 | 218.86 | 66.85 | 73 | 70.92 | 118.73 | 1.62 | 25.81 | 12.26 | 0.0242 | 15.97 | 412.16 |
| Normal | F | 21 | 64.88 | 248.28 | 76.83 | 76.07 | 86.05 | 144.91 | 1.51 | 25.19 | 12.39 | 0.0197 | 16.64 | 419.18 |
| average | | 31.632 | 69.400 | 273.009 | 71.090 | 78.811 | 86.675 | 150.345 | 1.865 | 35.958 | 10.971 | 0.027 | 19.360 | 757.701 |
| average dev | | 8.494 | 12.658 | 45.118 | 10.490 | 10.633 | 19.504 | 30.568 | 0.322 | 10.086 | 1.366 | 0.005 | 4.500 | 368.848 |
| | | | | | | | | | | | | | | |
| | | | | | | | | | | | | | | |
| | | | | | | | | | | | | | | |

**Table 2**

| Group | Age (y) Mean ± SD (Range) | Gender (n) Female / Male) | MMSE Score Mean ± SD (Range) |
|---|---|---|---|
| AD (n=20) | 74.0 ± 7.6 | 16 / 4 | 19.6 ± 6.4 |
| | (55-84) | | (7-28) |
| MD (n=20) | 67.7 ± 7.2 | 12 / 8 | 27.2 ± 2.0 |
| | (57-82) | | (21-30) |
| SCl (n=20) | 60.2 ± 10.4 | 8 / 12 | 28.7 ± 1.3 |
| | (40-74) | | (25-30) |

**Table 3**

| Gradient step | Time (minutes) | %A | %B | %C | %D |
|---|---|---|---|---|---|
| 1 | 0.0 | 99.0 | 0.0 | 0.0 | 1.0 |
| 2 | 6.0 | 98.0 | 0.0 | 0.0 | 2.0 |
| 3 | 8.0 | 90.0 | 6.0 | 2.0 | 2.0 |
| 4 | 12.0 | 80.0 | 14.0 | 3.0 | 3.0 |
| 5 | 18.0 | 20.0 | 74.0 | 3.0 | 3.0 |
| 6 | 19.0 | 20.0 | 74.0 | 3.0 | 3.0 |
| 7 | 20.0 | 95.0 | 0.0 | 2.0 | 3.0 |
| 8 | 22.0 | 99.0 | 0.0 | 0.0 | 1.0 |
| 9 | 30.0 | 99.0 | 0.0 | 0.0 | 1.0 |

**Table 4**

| | AD | MD | SCl | Kruskal-Wallis |
|---|---|---|---|---|
| TRP [µg/ml] | 10.7 ±2.1 | 10.9 ± 2.4 | 12.6 ± 3.0 | X² = 5.507 |
| | | | | p =0.064 |
| KYN [ng/ml] | 550.5 ± 232.6 | 466.2 ± 120.2 | 487.3 ± 123.3 | X = 1.536 |
| | | | | p =0.464 |
| KYNA [ng/ml] | 11.1 ± 5.6 | 11.2 ± 6.0 | 10.8 ± 3.8 | X² =0.033 |
| | | | | p =0.984 |
| 3-HK [ng/ml] | 32.6 ± 13.0 | 16.9 ± 17.5 | 13.3 ± 10.5 | X² = 20.281 |
| | | | | p <0.0001 |
| 3-HK : TRP ratio | 3.16 ± 1.54 | 1.81 ± 2.61 | 1.11 ± 0.90 | X² = 21.919 |
| | | | | P <0.0001 |

## Claims

1. A method for detecting depression comprising the steps of measuring the concentration of at least one *in vivo* degradation product of tryptophan, selected from the group consisting of 3-hydroxykynurenine, kynurenic acid, and kynurenine, in a body fluid selected from the group consisting of whole blood, serum, plasma, urine, saliva and cerebrospinal fluid (CSF), obtained from an individuum; and at least one of the following steps (a) to (c):
(a) determining the neuroprotective ratio by dividing the value of the concentration of kynurenic acid by the value of the concentration of kynurenine in said body fluid;
(b) determining the neuroprotective index by dividing the square value of the concentration of kynurenic acid by the value of the concentration of kynurenine in said body fluid;
(c) determining the ratio by dividing the value of the concentration of 3-hydroxykynurenine by the value of the concentration of kynurenic acid in said body fluid;
and assessing the depression.

2. The method according to claim 1, wherein an individuum having a depression is **characterized by** a neuroprotective ratio in the body fluid of 0 to 18.

3. The method according to claim 1, wherein an individuum having a depression is **characterized by** a neuroprotective index of 0 to 700.

4. The method according to claim 1, wherein an individuum having a depression is **characterized by** a ratio determined in step (c) which is two or higher:

5. A method for detecting depression comprising the step of combining at least two values selected from the group consisting of the concentration of kynurenic acid, the neuroprotective ratio, the ratio determined by dividing the value of the concentration of 3-hydroxykynurenine by the value of the concentration of kynurenic acid in a body fluid, and the neuroprotective index of a body fluid.

6. The use of a neuroprotective ratio determined by dividing the value of the concentration of kynurenic acid by the value of the concentration of kynurenine in a body fluid as a predictive marker for the detection of depression.

7. The use of a neuroprotective index determined by dividing the square value of the concentration of kynurenic acid by the value of the concentration of kynurenine in a body fluid as a predictive marker for the detection of depression.

8. The use of a ratio determined by dividing the value of the concentration of 3-hydroxykynurenine by the value of the concentration of kynurenic acid in a body fluid as a predictive marker for the detection of depression.

9. The use of a combination of at least two values selected from the group consisting of the concentration of kynurenic acid, the neuroprotective ratio, the ratio determined by dividing the value of the concentration of 3-hydroxykynurenine by the value of the concentration of kynurenic acid and the neuroprotective index of a body fluid as predictive markers for the detection of depression.

## Patentansprüche

1. Verfahren zum Nachweis von Depression, umfassend die Schritte des Messens der Konzentration von mindestens einem *in vivo* Abbauprodukt von Tryptophan, ausgewählt aus der Gruppe, bestehend aus 3-Hydroxykynurenin, Kynurensäure und Kynurenin, in einer Körperflüssigkeit, ausgewählt aus der Gruppe, bestehend aus Vollblut, Serum, Plasma, Urin, Speichel und cerebrospinaler Flüssigkeit (CSF), die von einem Individuum erhalten wird, und mindestens einen der folgenden Schritte (a) bis (c):
(a) Bestimmen des neuroprotektiven Verhältnisses durch Dividieren des Werts der Konzentration von Kynurensäure durch den Wert der Konzentration von Kynurenin in der Körperflüssigkeit,
(b) Bestimmen des neuroprotektiven Index durch Dividieren des quadratischen Werts der Konzentration von Kynurensäure durch den Wert der Konzentration von Kynurenin in der Körperflüssigkeit,
(c) Bestimmen des Verhältnisses durch Dividieren des Wertes der Konzentration von 3-Hydroxykynurenin durch den Wert der Konzentration von Kynurensäure in der Körperflüssigkeit,
und Einschätzen der Depression.

2. Verfahren nach Anspruch 1, wobei ein Individuum, das eine Depression aufweist, durch ein neuroprotektives Verhältnis in der Körperflüssigkeit von 0 bis 18 **gekennzeichnet** ist.

3. Verfahren nach Anspruch 1, wobei ein Individuum, das eine Depression aufweist, durch einen neuroprotektiven Index von 0 bis 700 **gekennzeichnet** ist.

4. Verfahren nach Anspruch 1, wobei ein Individuum, das eine Depression aufweist, durch ein in Schritt (c) bestimmtes Verhältnis, das zwei oder höher ist, **gekennzeichnet** ist.

5. Verfahren zum Nachweis von Depression, umfassend den Schritt des Kombinierens mindestens zweier Werte, ausgewählt aus der Gruppe, bestehend aus der Konzentration von Kynurensäure, dem neuroprotektiven Verhältnis, dem Verhältnis, das durch Dividieren des Wertes der Konzentration von 3-Hydroxykynurenin durch den Wert der Konzentration von Kynurensäure in einer Körperflüssigkeit bestimmt wird, und dem neuroprotektiven Index einer Körperflüssigkeit.

6. Verwendung eines neuroprotektiven Verhältnisses, bestimmt durch Dividieren des Werts der Konzentration von Kynurensäure durch den Wert der Konzentration von Kynurenin in einer Körperflüssigkeit als prognostischen Marker für den Nachweis von Depression.

7. Verwendung eines neuroprotektiven Index, bestimmt durch Dividieren des quadratischen Werts der Konzentration von Kynurensäure durch den Wert der Konzentration von Kynurenin in einer Körperflüssigkeit als prognostischen Marker für den Nachweis von Depression.

8. Verwendung eines Verhältnisses, bestimmt durch Dividieren des Werts der Konzentration von 3-Hydroxykynurenin durch den Wert der Konzentration von Kynurensäure in einer Körperflüssigkeit als prognostischen Marker für den Nachweis von Depression.

9. Verwendung einer Kombination von mindestens zwei Werten, ausgewählt aus der Gruppe, bestehend aus der Konzentration von Kynurensäure, dem neuroprotektiven Verhältnis, dem Verhältnis, das durch Dividieren des Werts der Konzentration von 3-Hydroxykynurenin durch den Wert der Konzentration von Kynurensäure bestimmt wird, und dem neuroprotektiven Index einer Körperflüssigkeit als prognostischen Marker für den Nachweis von Depression.

## Revendications

1. Procédé de détection de la dépression, comprenant les étapes consistant à mesurer la concentration d'au moins un produit de dégradation *in vivo* du tryptophane, choisi dans le groupe comprenant la 3-hydroxykynurénine, l'acide kynurénique et la kynurénine, dans un fluide corporel choisi dans le groupe comprenant le sang entier, le sérum, le plasma, l'urine, la salive et le liquide céphalorachidien (LCR) obtenu chez un individu ; et au moins l'une des étapes (a) à (c) suivantes :
(a) détermination du rapport neuroprotecteur en divisant la valeur de la concentration d'acide kynurénique par la valeur de la concentration de kynurénine dans ledit fluide corporel ;
(b) détermination de l'indice neuroprotecteur en divisant la valeur au carré de la concentration de l'acide kynurénique par la valeur de la concentration de kynurénine dans ledit fluide corporel ;
(c) détermination du rapport en divisant la valeur de la concentration de 3-hydroxykynurénine par la valeur de la concentration d'acide kynurénique dans ledit fluide corporel ;
et l'évaluation de la dépression.

2. Procédé selon la revendication 1, dans lequel un individu ayant une dépression est **caractérisé par** un rapport neuroprotecteur dans le fluide corporel de 0 à 18.

3. Procédé selon la revendication 1, dans lequel un individu ayant une dépression est **caractérisé par** un indice neuroprotecteur de 0 à 700.

4. Procédé selon la revendication 1, dans lequel un individu ayant une dépression est **caractérisé par** un rapport déterminé à l'étape (c) qui est de deux ou supérieur.

5. Procédé de détection de la dépression, comprenant l'étape consistant à combiner au moins deux valeurs choisies dans le groupe comprenant la concentration d'acide kynurénique, le rapport neuroprotecteur, le rapport déterminé en divisant la valeur de la concentration de la 3-hydroxykynurénine par la valeur de la concentration de l'acide kynurénique dans un fluide corporel et l'indice neuroprotecteur d'un fluide corporel.

6. Utilisation d'un rapport neuroprotecteur déterminé en divisant la valeur de la concentration d'acide kynurénique par la valeur de la concentration de kynurénine dans un fluide corporel en tant que marqueur prédictif pour la détection de la dépression.

7. Utilisation d'un indice neuroprotecteur déterminé en divisant la valeur au carré de la concentration d'acide kynurénique par la valeur de la concentration de kynurénine dans un fluide corporel en tant que marqueur prédictif pour la détection de la dépression.

8. Utilisation d'un rapport déterminé en divisant la valeur de la concentration de 3-hydroxykynurénine par la valeur de la concentration d'acide kynurénique dans un fluide corporel en tant que marqueur prédictif pour la détection de la dépression.

9. Utilisation d'une combinaison d'au moins deux valeurs choisies dans le groupe comprenant la concentration d'acide kynurénique, le rapport protecteur, le rapport déterminé en divisant la valeur de la concentration de 3-hydroxykynurénine par la valeur de la concentration d'acide kynurénique et l'indice neuroprotecteur d'un fluide corporel en tant que marqueurs prédictifs pour la détection de la dépression.
